# EUROPEAN PATENT APPLICATION

(11) **EP 4 382 526 A1**
(43) Date of publication of application: **12.06.2024**
(21) Application number: 22852367.6
(22) Date of filing: 05.08.2022
(51) Int. Cl.: C07D 409/14, C07D 417/06, C07D 417/14, C09K 11/06, C12Q 1/68

(54) **FLUORESCENT DYE, AND PREPARATION METHOD THEREFOR AND USE THEREOF**

(30) Priority: 06.08.2021 CN 202110903874
(71) Applicant: Fluorescence Diagnosis (shanghai) Biotech Company Ltd., Shanghai 200231 (CN)
(72) Inventor: ZHANG, Dasheng, Shanghai 200030 (CN)
(74) Representative: Bandpay & Greuter
(86) International application number: PCT/CN2022/110699
(87) International publication number: WO 2023/011656

(57) **Abstract**

A fluorescent dye as represented by formula (I), and a preparation method therefor and the use thereof. The fluorescent dye comprises an electron donor part D, a conjugate system E and an electron acceptor part A. The fluorescent dye with a long-wavelength emission capability has low background fluorescence, good water solubility, a sensitive viscosity response, and an adjustable wavelength.

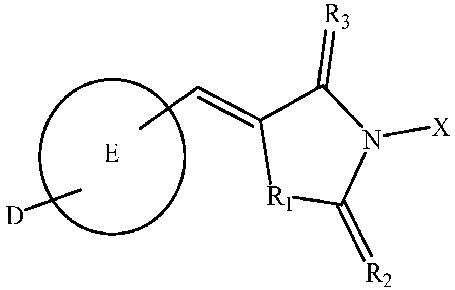

## Description

### Technical Field

The present disclosure relates to the technical field of fluorescent dyes, and in particular relates to a fluorescent dye having low background fluorescence, good water solubility, a sensitive viscosity response, and an adjustable molecular structure, and a preparation method therefor and use thereof.

### Background

Cell viscosity is one of the major parameters affecting the diffusion rate of a substance. By affecting the mobility of the substance, it has great influences on the transport, signal transduction, recognition and biomacromolecule interaction thereof. At the same time, the change of intracellular viscosity, to some extent, is related to atherosclerosis, diabetes, Alzheimer's disease and tumor, and is a potential indicator of these diseases. Therefore, detecting intracellular viscosity is of great significance. Due to its unique advantages of being sensitive, immediate, in-situ, and visible or the like, the fluorescence method is an important way to test intracellular micro-viscosity. Viscosity of a macroscopically large-volume viscous fluid can be measured with a rotor viscometer, a falling ball viscometer, etc. Different from this, intracellular viscosity is usually measured with a molecular rotor-based fluorescent probe. Molecular rotors are a special class of fluorescent molecules, and the energy loss thereof is caused by non-radiative transitions in an excited state and varies with viscosity changes. Twists of molecular rotors will be hindered when the viscosity or surrounding rigidity increases, and the energy loss caused by non-radiative transition of molecular rotors will decrease, which is specifically expressed to be the increase of fluorescence intensity of molecular rotors. Hence, the fluorescence intensity of molecular rotors can directly reflect the viscosity of the intracellular microenvironment.

In addition to the detection of intracellular micro-viscosity, fluorescence molecules of viscosity-responsive molecular rotors are frequently used for designing fluorogenic probes. This is mainly because the twists and rotation of fluorescence molecules of molecular rotors are limited (equivalent to increase of viscosity) after binding to proteins, nucleic acids or other biomolecules, and the fluorescence is lighted, while unbound molecules are in a twisted state and do not fluoresce, as a result, fluorescent lighting of biomolecules with low background and high specificity is realized. Fluorescence imaging of biomolecules with low abundance in living organisms requires a lower background while a high signal-to-noise ratio is ensured. For instance, the widely used thiazole orange dye has good signal-to-noise ratio during labeling of large molecular proteins and tRNA, but shows relatively strong background fluorescence, so it is badly limited during labeling of some small biomolecules with low abundance. Therefore, it is extremely important to develop fluorescent dye molecules with low background, sensitivity to viscosity response and high signal-to-noise ratio, and they will be widely applied to more fields.

Following are main causes for high background of various fluorescent probes. Firstly, fluorescence of fluorescent probes, which are normally on, cannot be quenched and thus causes strong fluorescent background, e.g., coumarin, fluorescein, Rhodamine and other traditional fluorescent dyes per se fluoresce brightly in water and need to be washed away for imaging after binding to a target substance, but they always cannot be thoroughly removed and thus cause strong background fluorescence. Secondly, background fluorescence occurs if quenching of quenchable fluorescent probes is not complete, for example, after fluorescence of Rhodaming is quenched according to PET and FRET by typical quenching groups, strong background fluorescence will occur during imaging of RNA because of incomplete quenching. Lastly, environment-responsive fluorescent probes are lipophilic because of poor water solubility thereof, and background fluorescence thus occurs. Environment-responsive fluorescent molecules per se do not fluoresce in water, and the fluorescent light will be lighted when they bind to the target. However, if the water solubility of a fluorescent probe is so poor that the environment-responsive fluorescent molecules bind to the cell membrane structure based on the principle of similarity and compatibility, some fluorescent light may be activated and the background problem may occur. Therefore, as for a viscosity-responsive fluorescent probe, enhancement of the water solubility of the probe is a key factor to solve the problem of background fluorescence.

The largest advantage of fluorescent dye as a fluorescent probe for fluorescent proteins is that micromolecular fluorescent dye has an adjustable structure, and the wavelength is thus adjustable for realizing biomolecule labeling in different colors. The micromolecular fluorescent probes have adjustable structures, but many of them require lots of procedures in organic synthesis and many molecular synthesis yields are not high, so a relatively high price is needed. Furthermore, a long wavelength is apparently advantageous for a fluorescent dye, but long-wavelength molecules in the structure of existing dyes generally have relatively large conjugate, which generally indicates poor water solubility of a compound and causes the problem of background fluorescence. If the wavelength can be changed significantly and can be moved towards a long wavelength by means of changing only one chemical element on the dye molecule, it can be ensured that the fluorescence molecular structure is small and the wavelength becomes longer, thereby ensuring the water solubility of dye molecules while elongating the long wavelength of dye molecules, which is of great significance to biological imaging.

### Summary

The object of the present disclosure is to provide a fluorescent dye with low background fluorescence, good water solubility, a sensitive viscosity response, and an adjustable wavelength.

A first aspect of the present disclosure is to provide a fluorescent dye, comprising: an electron donor part D, a conjugate system E and an electron acceptor part A, wherein the fluorescent dye is represented by Formula (I) 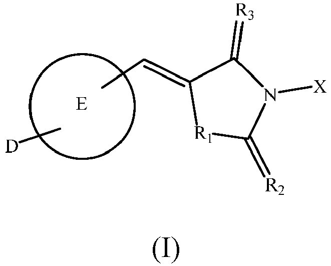
the electron donor part-D is (X₁)(X₂)N-, X₁ and X₂ are independently selected from hydrogen, alkyl and modified alkyl; X₁ and X₂ are optionally interconnected, and form an aliphatic heterocycle with N atoms;
the conjugate system E is formed by at least one conjugate connection selected from a sub-aromatic ring and a sub-aromatic heterocycle, wherein each hydrogen atom contained in the conjugate system E is optionally and independently substituted by alkyl groups;
X₁ and X₂ in the electron donor part D- optionally and independently form, together with N atoms, an aliphatic heterocycle with the conjugate system E;
the electron acceptor part optionally forms a ring structure represented by Formula (I-1): 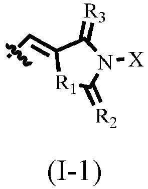 wherein:
   R₁ is independently selected from -O-, -S- and -(NRₐ)-, and Rₐ is selected from hydrogen and alkyl;
   R₂ is independently selected from =O, =S, and =NH;
   R₃ is independently selected from =O, =S and =NH; and
   X is independently selected from hydrogen, alkyl or modified alkyl;
   wherein: the "alkyl" is C₁-C₃₀ straight or branched alkyl; preferably, the "alkyl" is C₁-C₁₀ straight or branched alkyl; preferably, the "alkyl" is C₁-C₇ straight or branched alkyl; preferably, the "alkyl" is C₁-C₅ straight or branched alkyl; preferably, the "alkyl" is selected from methyl, ethyl, n-propyl, isopropyl, n-butyl, isobutyl, tert-butyl, sec-butyl, n-pentyl, 1-methylbutyl, 2-methylbutyl, 3-methylbutyl, isopentyl, 1-ethylpropyl, neopentyl, n-hexyl, 1-methylpentyl, 2-methylpentyl, 3-methylpentyl, isohexyl, 1,1-dimethylbutyl, 2,2-dimethylbutyl, 3,3-dimethylbutyl, 1,2-dimethylbutyl, 1,3-dimethylbutyl, 2,3-dimethylbutyl, 2-ethylbutyl, n-heptyl, 2-methylhexyl, 3-methylhexyl, 2,2-dimethylpentyl, 3,3-dimethylpentyl, 2,3-dimethylpentyl, 2,4-dimethylpentyl, 3-ethylpentyl or 2,2,3-trimethylbutyl;
   the "modified alkyl" is a group obtained by replacing any carbon atom in the alkyl with at least one group selected from halogen atom, -O-, -OH, -CO-, -SO₂-, -(S=O)-, primary amino group, secondary amino group and tertiary amino group, and the modified alkyl has 1 to 30 carbon atoms (optionally, the modified alkyl has 1 to 12 carbon atoms; optionally, the modified alkyl has 1 to 10 carbon atoms; optionally, the modified alkyl has 1 to 8 carbon atoms; optionally, the modified alkyl has 1 to 5 carbon atoms; optionally, the modified alkyl has 1 to 2 carbon atoms),
   the replacement of carbon atoms refers to that carbon atoms or the carbon atoms and hydrogen atoms thereon together are replaced by corresponding groups;
   the "aliphatic heterocycle" is a saturated or unsaturated 4- to 10-membered monocyclic or polycyclic aliphatic heterocycle containing at least one heteroatom of N, O or S on the ring, and when the aliphatic heterocycle contains an S atom, it is -S-, -SO- or -SO₂-;the aliphatic heterocycle is optionally substituted by alkyl;
   the "sub-aromatic ring" is a 6- to 13-membered monocyclic or fused dicyclic or fused polycyclic sub-aromatic group;
   the "sub-aromatic heterocycle" is a 5- to 12-membered monocyclic or fused dicyclic or fused polycyclic sub-aromatic heterocyclic group containing at least one heteroatom selected from N, O, S or Si on the ring;
   the "halogen atom" is respectively and independently selected from F, Cl, Br and I;
   the "primary amino group" is an RNH₂ group;
   the "secondary amino group" is an RNHR' group;
   the "tertiary amino group" is an RNR'R" group;
   each R, R' and R" respectively and independently is a single bond, alkyl, alkylene, modified alkyl or modified alkylene;
   the "alkylene" is a C₁-C₁₀ straight or branched alkylene, and optionally is a C₁-C₆ straight or branched alkylene; and
   the "modified alkylene" is a group obtained by replacing any carbon atom of C₁-C₁₀ (optionally C₁-C₆) alkyl or alkylene with a group selected from -O-, -OH, -CO-, -CS- and -(S=O)-; the replacement of carbon atoms refers to that carbon atoms or the carbon atoms and hydrogen atoms thereon together are replaced by corresponding groups;
   optionally, the modified alkyl or modified alkylene respectively and independently is a group containing at least one group selected from -OH, -O-, -NH₂, ethylene glycol unit (-(CHzCHzO)n-), C₁-C₈ alkyl, C₁-C₈ haloalkyl (preferably C₂-C₆ haloalkyl), C₁-C₈ alkyl carboxyl (preferably C₂-C₆ alkyl carboxyl) -SO₂-NH-, halogen atom, cyano group and nitryl, wherein n is 1 to 30, more preferably 1 to 10; and more preferably 1 to 2;
   optionally, the C₁-C₈ alkyl is methyl, ethyl, propyl or isopropyl; the C₁-C₈ alkoxy is methoxy, ethoxy, propoxy or isopropoxy, the C₁-C₈ acyl oxygroup is acetoxyl group, ethyl, propyl or isopropyl, and the C₁-C₈ haloalkyl is trifluoromethyl, chloromethyl or bromomethyl;
   optionally, the aliphatic heterocycle is selected from morpholine, thiomorpholine and tetrahydropyridine;
   optionally, X₁ and X₂ respectively and independently are C₁₋₁₀ straight or branched alkyl substituted by one or more groups selected from hydroxyl, cyano, halogen and carboxyl;
   optionally, the conjugate system E is a structure selected from following Formulae (I-2-1) to (I-2-20): 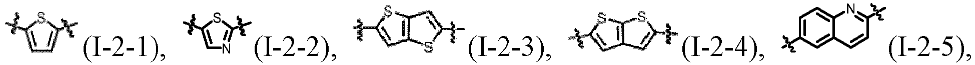 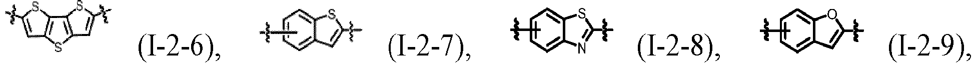 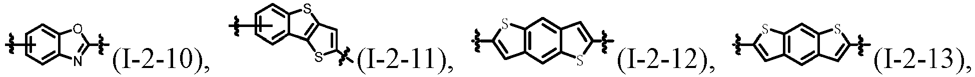 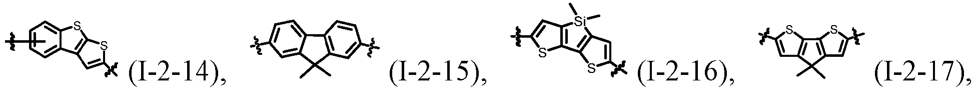 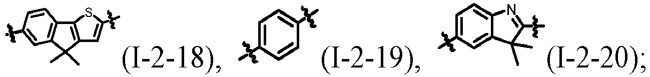
   or, the conjugate system E and (X₁)(X₂)N- form a aliphatic heterocycle as represented by Formulae (I-3-1) to (I-3-4): 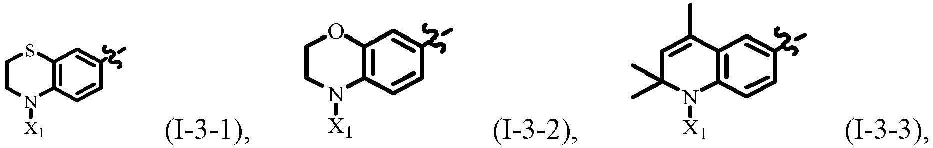 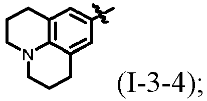
   optionally, the electron acceptor part A is one selected from Formulae (I-4-1) to (I-4-43): 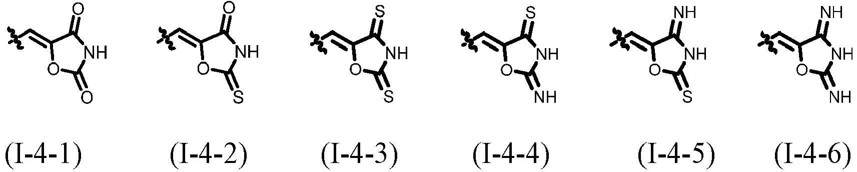 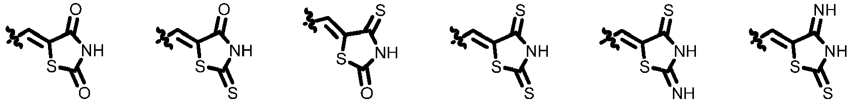 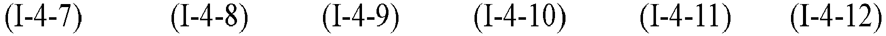 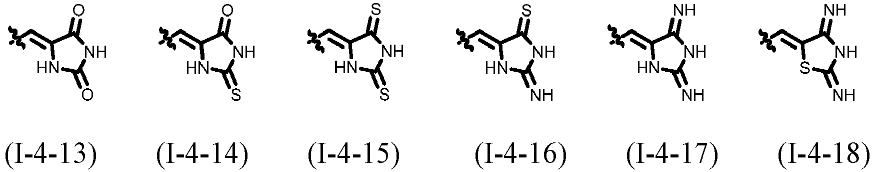 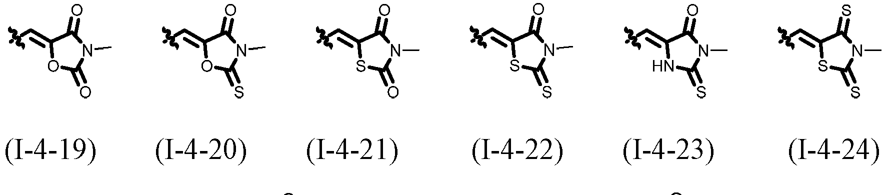 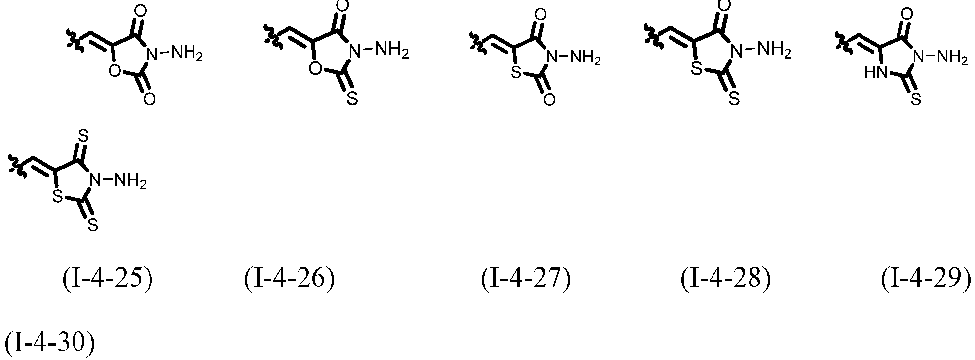 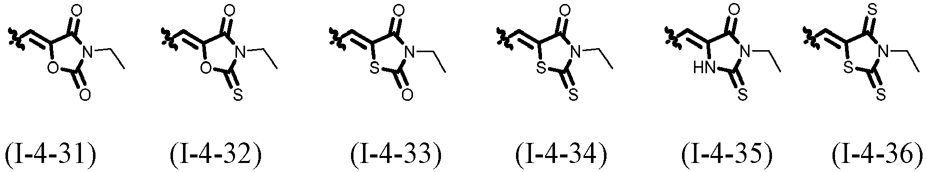 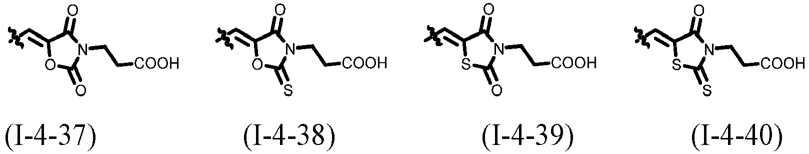 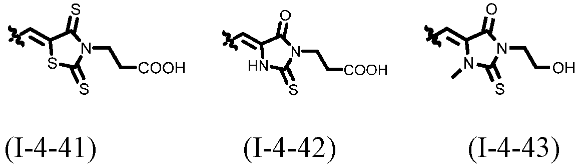
   optionally, the fluorescent dye represented by formula (I) is selected from the compounds represented by the formulae below:

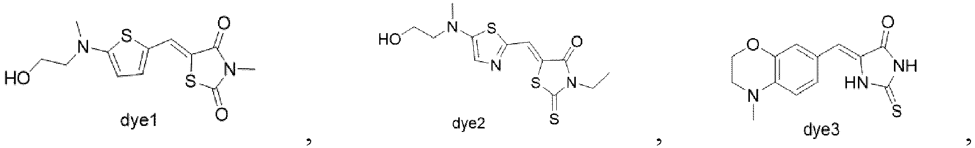

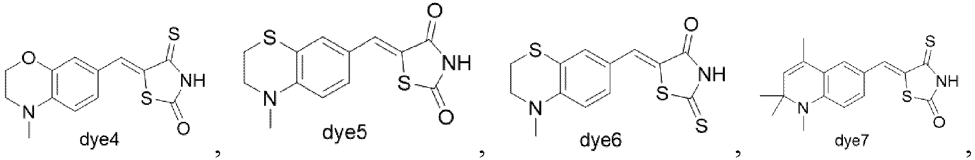

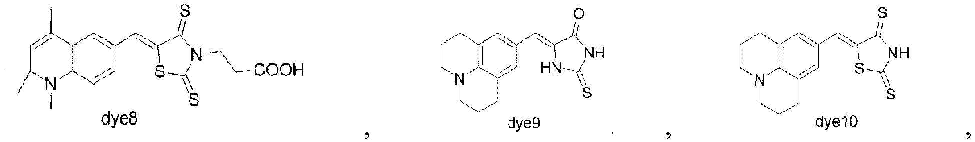

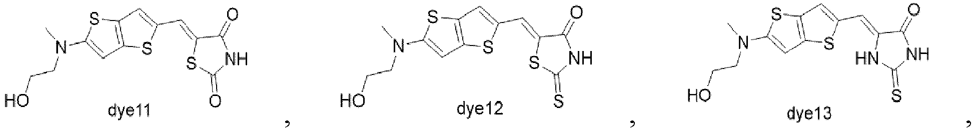

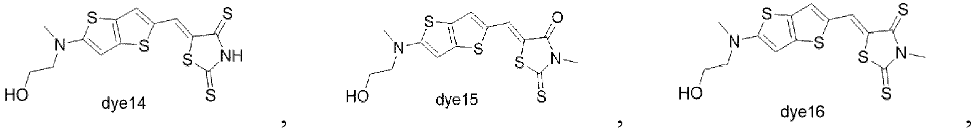

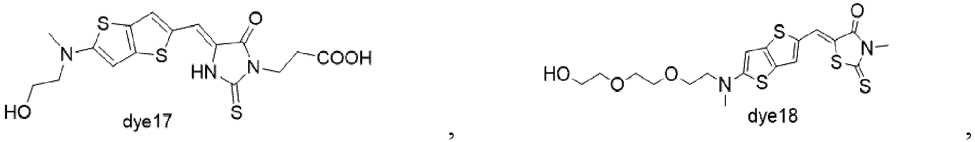

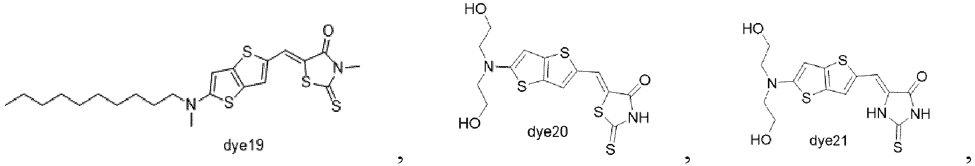

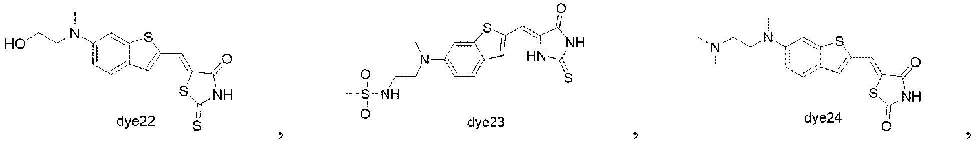

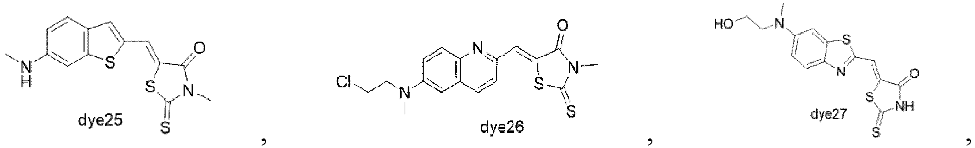

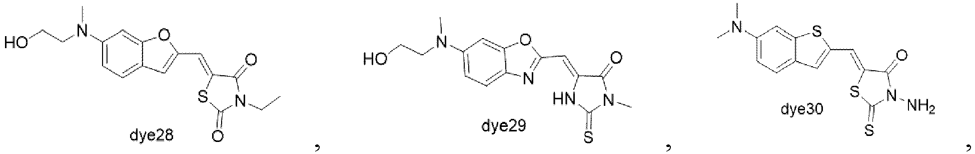

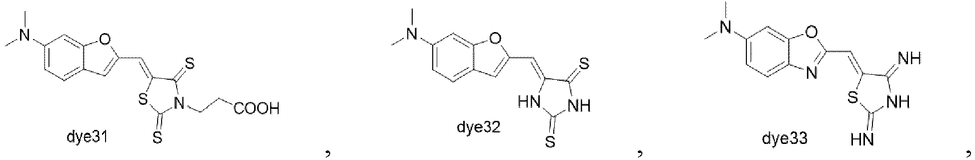

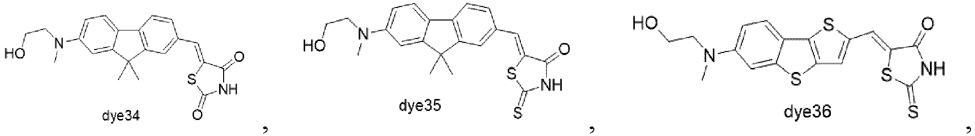

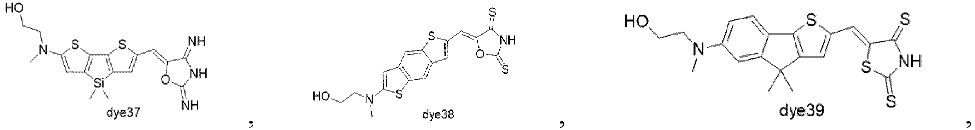

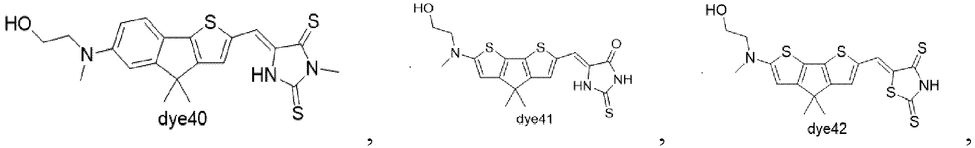

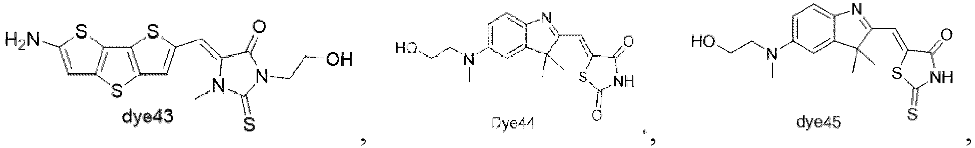

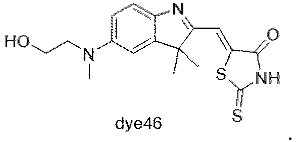

A second aspect of the present disclosure is to provide a method for preparing the fluorescent dye, including a synthetic reaction step of producing the fluorescent dye represented by Formula (I) via reaction of a compound represented by Formula (a) with a compound represented by Formula (b):

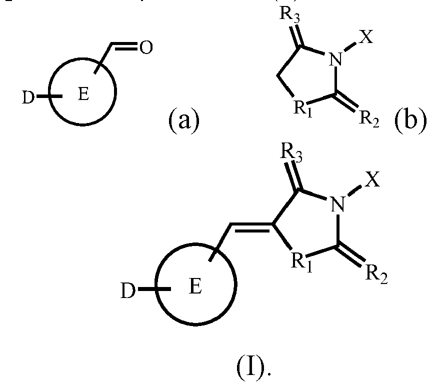

A third aspect of the present disclosure is to provide use of the fluorescent dye in viscosity testing, protein fluorescent labeling, nucleic acid fluorescent labeling, protein quantification or detection, or nucleic acid quantification or detection.

A fourth aspect of the present disclosure is to provide use of the fluorescent dye in preparing reagents for viscosity testing, protein fluorescent labeling, nucleic acid fluorescent labeling, protein quantification or detection, or nucleic acid quantification or detection.

A fifth aspect of the present disclosure is to provide a fluorescent activated and lighted probe comprising the fluorescent dye.

A sixth aspect of the present disclosure is to provide use of the fluorescent activated and lighted probe in protein fluorescent labeling, nucleic acid fluorescent labeling, protein quantification or detection, or nucleic acid quantification or detection.

A seventh aspect of the present disclosure is to provide use of the fluorescent activated and lighted probe in preparing reagents for protein fluorescent labeling, nucleic acid fluorescent labeling, protein quantification or detection, or nucleic acid quantification or detection.

The fluorescent dye obtained in the present disclosure is capable of long wavelength emission (with a longest emission wavelength of 800 nm). The fluorescence intensity thereof increases with environmental viscosity, the logarithm of the fluorescence intensity and that of the solvent viscosity are in a good linear relationship, and the relationship between the fluorescence intensity and the viscosity conforms to Hofmann equation and has a high slope, which means that the fluorescent dye is sensitive to viscosity, has high activation multiple and is insensitive to polarity changes.

The fluorescent dye of the present disclosure can be used for measuring viscosity of samples, such as for the testing of micro-viscosity. According to the embodiments of another aspect, the obtained fluorescent dye can be specifically combined with corresponding antibody, aptamer or amyloid, or bound to the protein tag or enzyme via a ligand or inhibitor, thereby obtaining a series of fluorescent activated and lighted probes used for fluorescent labeling, quantification or monitoring of proteins, enzymes or nucleic acids.

### Description of Drawings

FIG. 1 is a diagram showing the fluorescence emission intensity of dye7 (1 × 10⁻⁵M) under different viscosity conditions;
FIG. 2 is a diagram showing the linear relationship between the viscosity conditions and the fluorescence intensity of dye7 (1×10⁻⁵M);
FIG. 3 is a diagram showing the fluorescence emission intensity of dye9 (1×10⁻⁵M) under different viscosity conditions;
FIG. 4 is a diagram showing the linear relationship between the viscosity conditions and the fluorescence intensity of dye9 (1×10⁻⁵M);
FIG. 5 is a diagram showing the fluorescence emission intensity of dye12 (1×10⁻⁵M) under different viscosity conditions;
FIG. 6 is a diagram showing the linear relationship between the viscosity conditions and the fluorescence intensity of dye12 (1×10⁻⁵M);
FIG. 7 is a diagram showing the fluorescence emission intensity of dye25 (1×10⁻⁵M) under different viscosity conditions;
FIG.8 is a diagram showing the linear relationship between the viscosity conditions and the fluorescence intensity of dye25 (1×10⁻⁵M);
FIG. 9 is a diagram showing the fluorescence emission intensity of dye40 (1×10⁻⁵M) under different viscosity conditions;
FIG. 10 is a diagram showing the linear relationship between the viscosity conditions and the fluorescence intensity of dye40 (1×10⁻⁵M);
FIG. 11 is a diagram showing the fluorescence emission intensity of dye7 (1×10⁻⁵M) under different polar conditions;
FIG. 12 is a diagram showing the fluorescence emission intensity of dye9 (1×10⁻⁵M) under different polar conditions;
FIG. 13 is a diagram showing the fluorescence emission intensity of dye12 (1×10⁻⁵M) under different polar conditions;
FIG. 14 is a diagram showing the fluorescence emission intensity of dye25 (1×10⁻⁵M) under different polar conditions;
FIG. 15 is a diagram showing the fluorescence emission intensity of dye40 (1×10⁻⁵M) under different polar conditions;
FIG. 16 is a diagram showing the emission spectra contrast of dye5 and dye6;
FIG. 17 is a diagram showing the emission spectra contrast of dye11, dye12 and dye14;
FIG. 18 is a diagram showing the emission spectra contrast of dye12 and dye 13;
FIG. 19 is the emission spectrum of dye39;
FIG. 20 is the emission spectrum of dye42;
FIG. 21 is a diagram showing the background fluorescence contrast of dye1 and dye48 in PBS solution;
FIG. 22 is a diagram showing the background fluorescence contrast of dye22 and dye49 in PBS solution;
FIG. 23 is a diagram showing the background fluorescence contrast of dye40 and dye50 in PBS solution;
FIG. 24 is a diagram showing the background fluorescence contrast of dye9, dye10 and dye47 in PBS solution;
FIG. 25 is a diagram showing use of dye1, dye12, dye25 and dye41 in labeling aptamers in cells;
FIG. 26 is a flow cytological map of dye3 binding to RNA aptamer;
FIG. 27 is a flow cytological map of dye 13 binding to RNA aptamer;
FIG. 28 is a flow cytological map of dye21 binding to RNA aptamer;
FIG. 29 is a flow cytological map of dye34 binding to RNA aptamer;
FIG. 30 is a flow cytological map of dye50 binding to RNA aptamer.

### Embodiments

Embodiments of the present disclosure will be explained in detail below. It should be understood that the embodiments described here are merely exemplary illustration of the present disclosure, rather than limits.

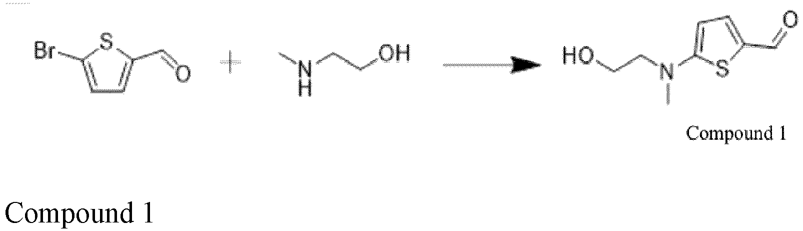

5-bromothiophene-2 methanal (0.5g, 2.6mmol) and 2-methylaminoethanol (0.78 g, 10.4 mmol) dissolved into 10 ml of water, and were heated to 100°C by means of oil bath under Ar atmosphere and were subject to react overnight; after the reaction, they were cooled to room temperature, extracted with dichloromethane for three times, washed with saturated salt solution, and dried with anhydrous sodium sulfate; the organic phase was dried by means of spinning, and the residue was subjected to column chromatography, as a result, 0.4 g of Compound 1 was obtained, and the yield was 84%. ¹H NMR (400 MHz, DMSO-*d*₆) δ9.40 (s, 1H), 7.65 (d, *J*=4.5 Hz, 1H), 6.12 (d, *J*=4.5 Hz, 1H), 3.62 (q, *J*=5.5 Hz, 2H), 3.47 (t, *J*=5.6 Hz, 2H), 3.09 (s, 3H).

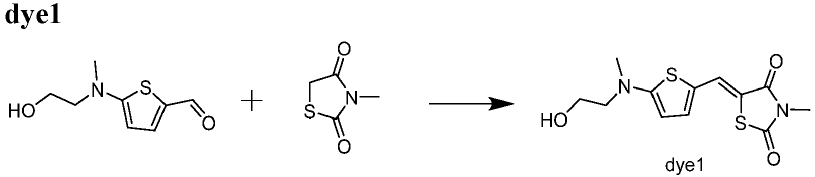

Compound 1 (0.2 g, 1.08 mmol) and 3-methylthiazolidine-2, 4-diketone (0.13 g, 1.30 mmol) dissolved into 50 ml of absolute ethyl alcohol, were added with a catalytic dose of piperidine, and were heated to 90°C by means of oil bath under Ar atmosphere were subject to reaction for 3 h; after the reaction, they were cooled to room temperature, and a large amount of solid precipitated in the system; the solid was filtered, and the filter cake was washed twice with cold alcohol and then was subject to vacuum drying, thereby obtaining 0.2 g of a yellow compound dye1, and the yield was 71.4%. ¹H NMR (400 MHz, DMSO-*d*₆) δ7.71 (s, 1H), 7.43 (d, *J*=4.4 Hz, 1H), 6.15 (d, *J*=4.4 Hz, 1H), 3.62 (q, *J*=*5.5* Hz, 2H), 3.46 (t, *J*=5.6 Hz, 2H), 3.20 (s, 3), 3.09 (s, 3H).

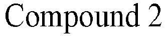

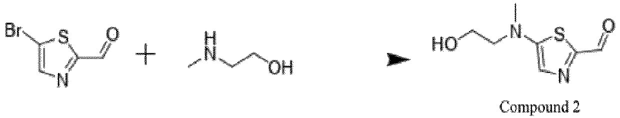

Compound 2 was synthesized with reference to the synthetic method of Compound 1, and the yield was 50%. ¹H NMR (400 MHz, DMSO-*d*₆) δ9.70 (s, 1H), 6.82 (s, 1H), 3.62 (q, *J*=5.5 Hz, 2H), 3.47 (t, *J*=5.6 Hz, 2H), 3.09 (s, 3H).

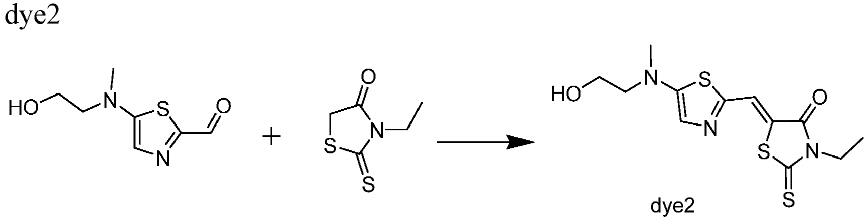

The dye2 was synthesized with reference to the synthetic method of dye1, and the yield was 76%. ¹H NMR (400 MHz, DMSO-*d*₆) δ9.70 (s, 1H), 6.82 (s, 1H), 4.10 (q, *J*=7.0 Hz, 4H), 3.62 (q, *J*=5.5 Hz, 2H), 3.47 (t, *J*=5.6 Hz, 2H), 3.09 (s, 3H), 1.2 (t, *J*=7.1 Hz 3H).

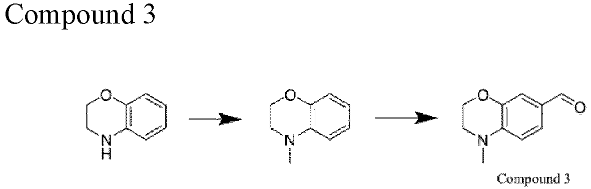

Benzomorpholine (0.30 g, 2 mmol) dissolved into 20 mL of DMF, and then cesium carbonate (0.78 g, 2.4 mmol) and iodomethane (0.31 g, 2.2 mmol) were added; they were heated to 65°C by means of oil bath under Ar atmosphere and were subject to reaction for 4 h; after the reaction, they were cooled to room temperature; the system was poured into 50 mL of water, and was extracted with dichloromethane for three times, 50 mL each time; the organic phase was combined and the solvent was dried by means of spinning and evaporation, as a result, 0.290 g of product was obtained after column chromatography separation, and the yield was 90.6%. ¹H NMR (400 MHz, DMSO-*d*₆) δ6.98-6.94 (m, 1H), 6.94-6.88 (m, 1H), 6.67 (dd, *J*=8.1, 1.2 Hz, 1H), 6.57 (td, *J*=7.5, 1.2 Hz, 1H), 3.38-3.32 (m, 2H), 3.05-2.80 (m, 2H), 2.69 (s, 3H).

A three-mouth flask was added with 10 ml of DMF and was cooled for 5 min by means of ice bath; 0.2 ml of phosphorus oxychloride was added dropwise, and was stirred for 1 h under the condition of ice bath; the compound dissolved into DMF, then the solution was added dropwise into the system and was stirred for 0.5 h under Ar atmosphere and the condition of ice bath; the system slowly recovered to room temperature, and was stirred for another 5 h; after the reaction, saturated sodium carbonate solution was added for regulation till pH=10.0, and the stirring was performed overnight at room temperature; the organic phase was separated the next day, and the aqueous phase was extracted three times with 50 ml of dichloromethane for three times; the organic phase was combined and was washed twice with saturated salt solution; the organic phase was dried with anhydrous sodium sulfate, the solvent was dried by means of spinning and evaporation, and the residue was separated with column chromatography separation, as a result, 0.25 g of yellow solid was obtained, and the yield was 74%. ¹H NMR (400 MHz, DMSO-*d*₆) δ10.26 (s, 1H), 6.98-6.94 (m, 1H), 6.94-6.88 (m, 1H), 6.57 (td, *J*=7.5, 1.2 Hz, 1H), 3.38-3.32 (m, 2H), 3.05-2.80 (m, 2H), 2.69 (s, 3H).

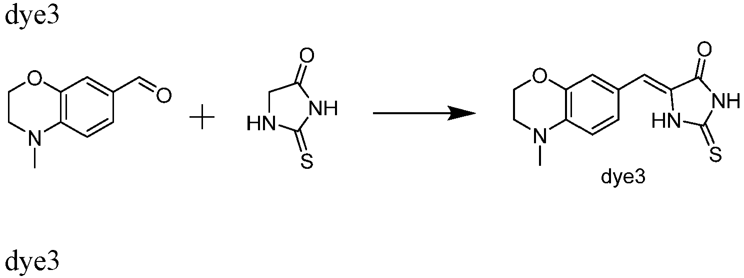

The dye3 was synthesized with reference to the synthetic method of dye1, and the yield was 82%. ¹H NMR (400 MHz, DMSO-*d*₆) δ11.71 (s, 1H), 11.56 (s, 1H), 6.98-6.94 (m, 1H), 6.94-6.88 (m, 1H), 6.65 (s, 1H), 6.57 (td, *J*=7.5, 1.2 Hz, 1H), 3.38-3.32 (m, 2H), 3.05-2.80 (m, 2H), 2.69 (s, 3H).

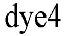

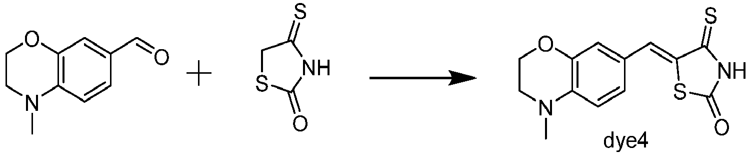

The dye4 was synthesized with reference to the synthetic method of dye1, and the yield was 70%. ¹H NMR (400 MHz, DMSO-*d*₆) δ13.81 (s, 1H), 6.98-6.94 (m, 1H), 6.94-6.88 (m, 1H), 6.65 (s, 1H), 6.57 (td, *J*=7.5, 1.2 Hz, 1H), 3.38-3.32 (m, 2H), 3.05-2.80 (m, 2H), 2.69 (s, 3H).

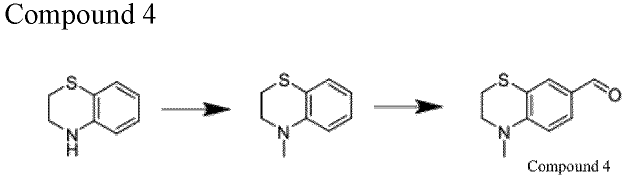

Compound 4 was synthesized with reference to the synthetic method of Compound 3, and the yield was 56%. ¹H NMR (400 MHz, DMSO-*d*₆) δ10.11 (s, 1H), 6.98-6.94 (m, 1H), 6.94-6.88 (m, 1H), 6.57 (td, *J*=7.5, 1.2 Hz, 1H), 3.57-3.42 (m, 2H), 3.13-3.00 (m, 2H), 2.87 (s, 3H).

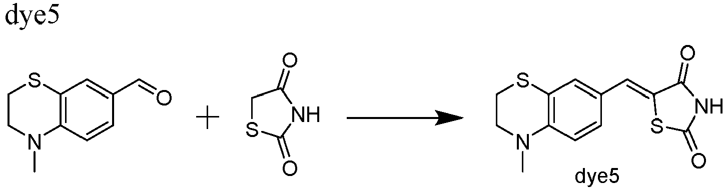

The dye5 was synthesized with reference to the synthetic method of dye1, and the yield was 65.8%. ¹H NMR (400 MHz, DMSO-*d*₆) δ11.75 (s, 1H), 7.42 (s, 1H), 7.38-7.15 (m, 2H), 6.82 (d, *J*=8.7 Hz, 1H), 3.76-3.57 (m, 2H), 3.13-3.05 (m, 2H), 3.03 (s, 3H).

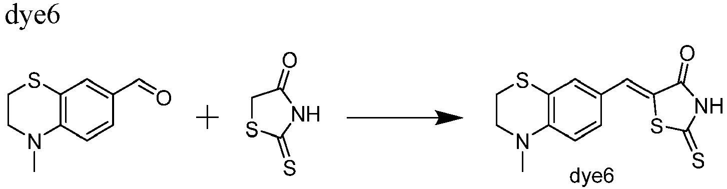

The dye6 was synthesized with reference to the synthetic method of dye1, and the yield was 75.8%. ¹H NMR (400 MHz, DMSO-*d*₆) δ13.15 (s, 1H), 7.42 (s, 1H), 7.38-7.15 (m, 2H), 6.82 (d, *J*=8.7 Hz, 1H), 3.76-3.57 (m, 2H), 3.13-3.05 (m, 2H), 3.03 (s, 3H).

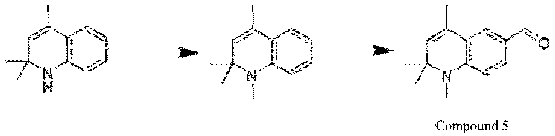

Compound 5 was synthesized with reference to the synthetic method of Compound 3, and the yield was 65.7%. ¹H NMR (400 MHz, DMSO-*d*₆) δ10.21 (s, 1H), 7.33 (dd, *J*=8.8, 2.2 Hz, 1H), 7.15 (d, *J*=2.2 Hz, 1H), 6.63 (d, *J*=8.8 Hz, 1H), 5.47 (d, *J*=1.5 Hz, 1H), 2.87 (s, 3H), 1.96 (d, *J*=1.4 Hz, 3H), 1.34 (s, 6H).

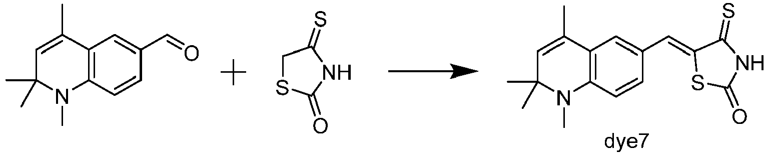

The dye7 was synthesized with reference to the synthetic method of dye1, and the yield was 84.2%. ¹H NMR (400 MHz, DMSO-*d*₆) δ13.86 (s, 1H), 7.41 (s, 1H), 7.33 (dd, *J*=8.8, 2.2 Hz, 1H), 7.15 (d, *J*=2.2 Hz, 1H), 6.63 (d, *J*=8.8 Hz, 1H), 5.47 (d, *J*=1.5 Hz, 1H), 2.87 (s, 3H), 1.96 (d, *J*=1.4 Hz, 3H), 1.34 (s, 6H).

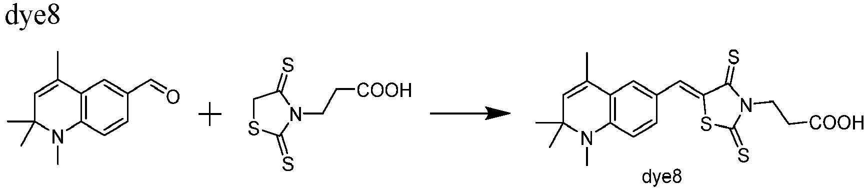

The dye8 was synthesized with reference to the synthetic method of dye1, and the yield was 80.3%. ¹H NMR (400 MHz, DMSO-*d*₆) δ7.41 (s, 1H), 7.33 (dd, *J*=8.8, 2.2 Hz, 1H), 7.15 (d, *J*=2.2 Hz, 1H), 6.63 (d, *J*=8.8 Hz, 1H), 5.47 (d, *J*=1.5 Hz, 1H), 4.40(m, 2H), 2.87 (s, 3H), 2.61(m, 2H), 1.96 (d, *J*=1.4 Hz, 3H), 1.34 (s, 6H).

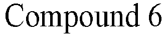

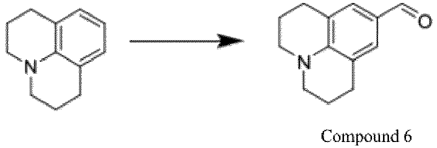

Compound 6 was synthesized with reference to the synthetic method of Compound 3, and the yield was 60.7%. ¹H NMR (400 MHz, DMSO-*d*₆) δ10.25 (s, 1H), 6.95 (s, 2H), 3.25 (dd, *J*=6.6, 4.9 Hz, 4H), 2.68 (t, *J*=6.3 Hz, 4H), 2.05-1.57 (m, 4H).

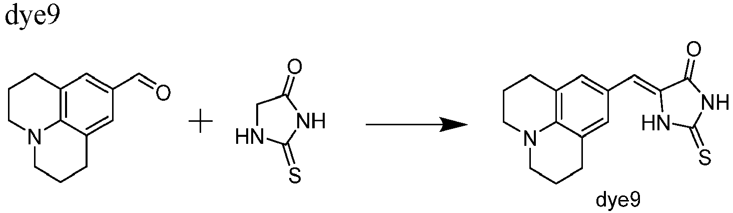

The dye9 was synthesized with reference to the synthetic method of dye1, and the yield was 88.6%. ¹H NMR (400 MHz, DMSO-*d*₆) δ12.21 (s, 1H), 7.31 (s, 1H), 6.95 (s, 2H), 3.25 (dd, *J*=6.6, 4.9 Hz, 4H), 2.68 (t, *J*=6.3 Hz, 4H), 2.05-1.57 (m, 4H).

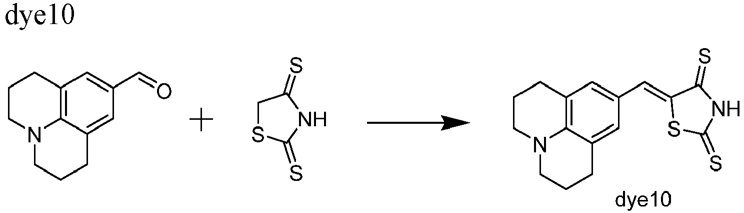

The dye10 was synthesized with reference to the synthetic method of dye1, and the yield was 82.4%. ¹H NMR (400 MHz, DMSO-*d*₆) δ19.21 (s, 1H), 7.31 (s, 1H), 6.95 (s, 2H), 3.25 (dd, *J*=6.6, 4.9 Hz, 4H), 2.68 (t, *J*=6.3 Hz, 4H), 2.05-1.57 (m, 4H).

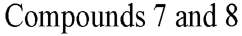

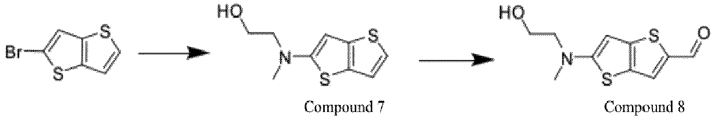

### Compound 7:

2-bromothieno[3,2-b]thiophene (0.438 g, 2 mmol) dissolved into 15 mL N-methyl-N-hydroxyethylamine, and then copper powder (6.4 mg, 0.01 mmol), copper iodide (19 mg, 0.01 mmol) and tripotassium phosphate (0.850 g, 4 mmol) were added; they were heated to 80°C by means of oil bath under Ar atmosphere and were subject to reaction overnight; after the reaction, they were cooled to room temperature; the system was poured into 50 mL of water, and was extracted with dichloromethane for three times, 50 mL each time; the organic phase was combined and the solvent was dried by means of spinning and evaporation, as a result, 0.362 g of yellow product was obtained after column chromatography separation, and the yield was 85%. ¹H-NMR (400 MHz, CDCl₃): δ=7.92 (s, 1H), 7.63 (d, 1H, *J*=5.2 Hz), 7.31 (d, 1H, *J*=5.2 Hz), 3.85 (t, 2H, *J*=5.6 Hz), 3.60 (t, 2H, *J*=5.6 Hz), 3.10 (s, 3H).

### Compound 8:

Compound 8 (0.426 g, 2 mmol) dissolved into 50 ml of anhydrous dichloromethane, 1 ml of triethylamine was added, and acetic oxide (0.3 ml, 3 mmol) was slowly and dropwise added under the condition of ice bath; after dropwise adding, the system slowly recovered to room temperature, and was stirred for 3 h; after the reaction, 100 ml of water was added, organic phase was separated, and aqueous phase was extracted twice with 50 ml of dichloromethane; the organic phase was combined and was dried with anhydrous sodium sulfate, and the solvent was dried by means of spinning and evaporation; and the residue needed no further purification and was directly used in the next step.

The above-mentioned residue was dissolved into 50 ml of dichloromethane, 5 ml of dimethylformamide was added, 2 ml of phosphorus oxychloride was added under the condition of ice bath, and stirring was performed under Ar atmosphere for 0.5 h; the system slowly recovered to room temperature, and was stirred for another 5 h; after the reaction, saturated sodium carbonate solution was added for regulation till pH=10.0, and the stirring was performed overnight at room temperature; the organic phase was separated the next day, and the aqueous phase was extracted with 50 ml of dichloromethane for three times; the organic phase was combined and was washed twice with saturated salt solution; the organic phase was dried with anhydrous sodium sulfate, the solvent was dried by means of spinning and evaporation, and the residue was separated with column chromatography separation, as a result, 0.285 g of yellow solid was obtained, and the yield was 59%. ¹H-NMR (400 MHz, CDCl₃): δ=10.01 (s, 1H), 7.92 (s, 1H), 7.63 (s, 1H), 3.85 (t, 2H, *J*=5.6 Hz), 3.60 (t, 2H, *J*=5.6 Hz), 3.10 (s, 3H).

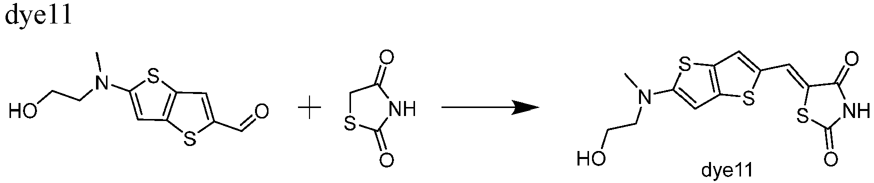

The dye11 was synthesized with reference to the synthetic method of dye1, and the yield was 78.4%. ¹H-NMR (400 MHz, CDCl₃): δ=12.01 (s, 1H), 7.57 (s, 1H), 7.92 (s, 1H), 7.63 (s, 1H), 3.85 (t, 2H, *J*=5.6 Hz), 3.60 (t, 2H, *J*=5.6 Hz), 3.10 (s, 3H).

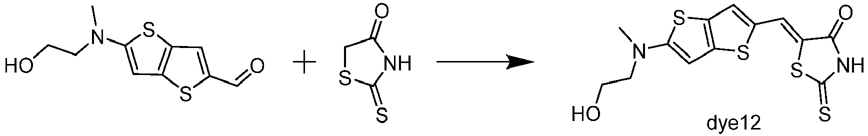

The dye12 was synthesized with reference to the synthetic method of dye1, and the yield was 78.3%. ¹H-NMR (400 MHz, CDCl₃): δ=13.25 (s, 1H), 7.57 (s, 1H), 7.92 (s, 1H), 7.63 (s, 1H), 3.85 (t, 2H, *J*=5.6 Hz), 3.60 (t, 2H, *J*=5.6 Hz), 3.10 (s, 3H).

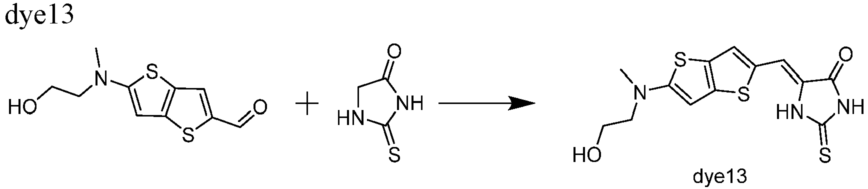

The dye13 was synthesized with reference to the synthetic method of dye1, and the yield was 87.1%. ¹H-NMR (400 MHz, CDCl₃): δ=11.35 (s, 1H), 11.25 (s, 1H), 7.87 (s, 1H), 7.92 (s, 1H), 7.63 (s, 1H), 3.85 (t, 2H, *J*=5.6 Hz), 3.60 (t, 2H, *J*=5.6 Hz), 3.10 (s, 3H).

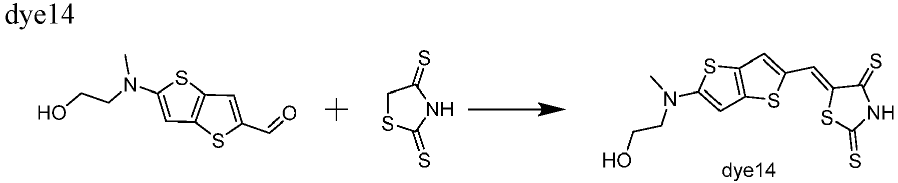

The dye14 was synthesized with reference to the synthetic method of dye1, and the yield was 87.1%. ¹H-NMR (400 MHz, CDCl₃): δ=18.35 (s, 1H), 6.87 (s, 1H), 7.92 (s, 1H), 7.63 (s, 1H), 3.85 (t, 2H, *J*=5.6 Hz), 3.60 (t, 2H, *J*=5.6 Hz), 3.10 (s, 3H).

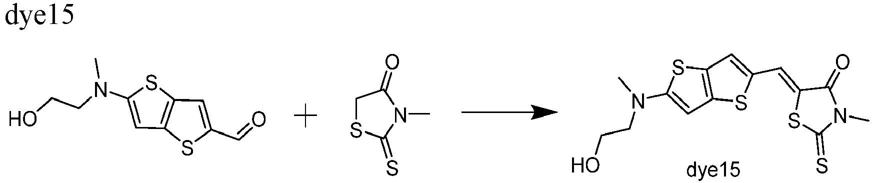

The dye15 was synthesized with reference to the synthetic method of dye1, and the yield was 84.7%. ¹H-NMR (400 MHz, CDCl₃): δ=7.42 (s, 1H), 7.92 (s, 1H), 7.63 (s, 1H), 3.85 (t, 2H, *J*=5.6 Hz), 3.60 (t, 2H, *J*=5.6 Hz), 3.34 (s, 3H), 3.10 (s, 3H).

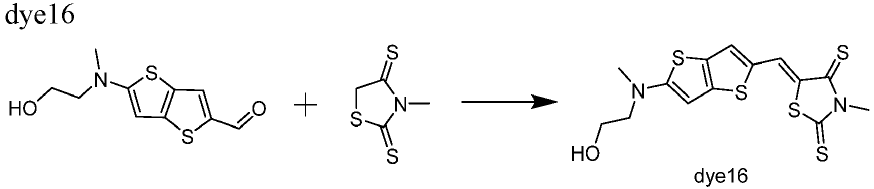

The dye16 was synthesized with reference to the synthetic method of dye1, and the yield was 82.1%. ¹H-NMR (400 MHz, CDCl₃): δ=6.72 (s, 1H), 7.92 (s, 1H), 7.63 (s, 1H), 3.85 (t, 2H, *J*=5.6 Hz), 3.60 (t, 2H, *J*=5.6 Hz), 3.52 (s, 3H), 3.10 (s, 3H).

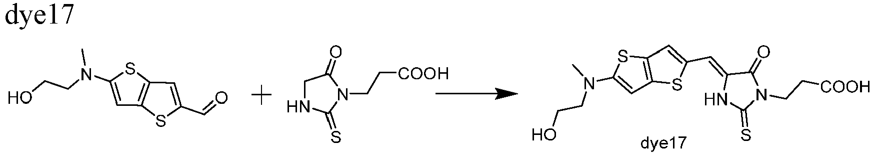

The dye17 was synthesized with reference to the synthetic method of dye1, and the yield was 82.7%. ¹H-NMR (400 MHz, CDCl₃): δ=12.03 (s, 1H), 11.41 (s, 1H), 6.72 (s, 1H), 7.92 (s, 1H), 7.63 (s, 1H), 4.40 (m, 2H), 3.85 (t, 2H, *J*=5.6 Hz), 3.60 (t, 2H, *J*=5.6 Hz), 3.52 (s, 3H), 3.10 (s, 3H), 2.62 (m, 2H).

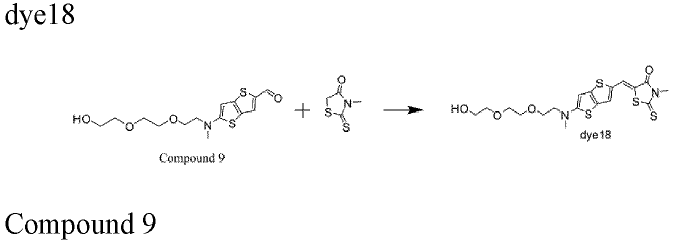

Compound 9 was synthesized with reference to the synthetic method disclosed in the following document (Organic&Biomolecular Chemistry, 7(1), 142-154; 2009). ¹H-NMR (400 MHz, CDCl₃): δ=10.05 (s, 1H), 7.57 (s, 1H), 7.92 (s, 1H), 3.95 (m, 4H), 3.45 (m, 8H), 3.10 (s, 3H).

Dye18 was synthesized with reference to the synthetic method of dye1, and the yield was 52.3%. ¹H-NMR (400 MHz, CDCl₃): δ=8.02 (s, 1H), 7.57 (s, 1H), 7.92 (s, 1H), 3.95 (m, 4H), 3.45 (m, 8H), 3.25 (s, 3H), 3.10 (s, 3H).

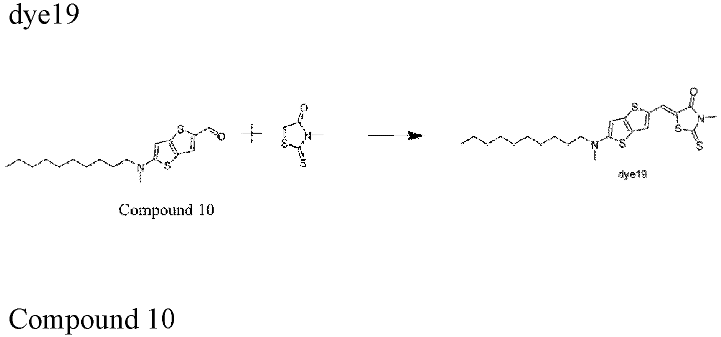

Compound 9 was synthesized with reference to the method disclosed in the following document (Journal of Organic Chemistry, 83(15), 8533-8542; 2018), and the yield was 52.1%. ¹H-NMR (400 MHz, CDCl₃): δ=10.15 (s, 1H), 7.57 (s, 1H), 7.92 (s, 1H), 3.10 (s, 3H), 3.08 (m,2H), 1.58-1.43 (m,18H).

The dye19 was synthesized with reference to the synthetic method of dye1, and the yield was 88.9%. ¹H-NMR (400 MHz, CDCl₃): δ=8.23 (s, 1H), 7.57 (s, 1H), 7.92 (s, 1H), 3.25 (s, 3H), 3.10 (s, 3H), 3.08 (m, 2H), 1.58-1.43 (m, 18H).

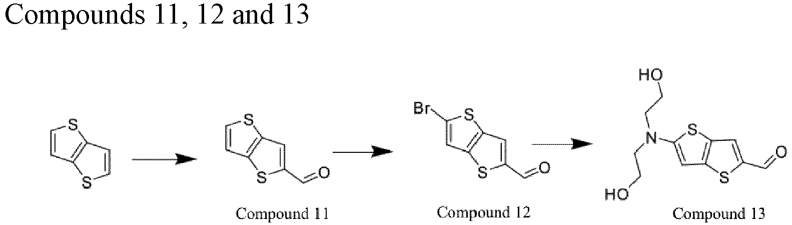

### Compound 11

To a three-mouth flask of 250 ml, thieno[3,2-b]thiophene (10 g, 71.31 mmol) was added and 120 ml of anhydrous tetrahydrofuran was added for dissolution, and then the three-mouth flask was placed at -78°C to be cooled; 49 ml of 1.6M n-butyllithium was added dropwise to the system; the temperature of -78°C was kept for 2h, then the refrigeration was turned off, and the temperature slowly recovered to room temperature; the stirring was performed overnight, and 40 ml of water was added under ice bath after the reaction so as to quench the reaction; the system was poured into 200 ml of dichloromethane and was extracted, the aqueous phase was extracted with dichloromethane for three times, the organic phase was dried with anhydrous sodium sulfate, the solvent was dried by means of spinning, and the residue was separated with column chromatography separation, as a result, 10.7 g of compound was obtained, and the yield was 90%. ¹H NMR (400 MHz, Chloroform-*d*) δ9.97 (s, 1H), 7.94 (s, 1H), 7.70 (d, *J*=5.3 Hz, 1H), 7.33 (d, *J*=5.3 Hz, 1H).

### Compound 12

Compound 11 (10 g, 59.5 mmol) dissolved in 120 ml of mixed solution of dry DMF and acetic acid (1:1), NBS (11.66 g, 65.5 mmol) was added, and was heated by means of oil bath under Ar atmosphere to 120°C overnight for refluxing; after the reaction, extraction was performed for three times with ethyl acetate and water, the organic phase was combined and dried, and then the solvent was dried by means of spinning, as a result, 11.2 g of product was obtained after the residual was subject to column chromatography, and the yield was 78%. ¹H NMR (400 MHz, Chloroform-*d*) δ10.01 (s, 1H), 7.70 (d, *J*=5.3 Hz, 1H), 7.33 (d, *J*=5.3 Hz, 1H).

### Compound 13

Compound 13 was synthesized with reference to the synthetic method of Compound 1, and the yield was 85%. ¹H NMR (400 MHz, DMSO-*d*₆) δ10.15 (s, 1H), 7.87 (s, 1H), 6.39 (s, 1H), 3.85 (t, 4H, *J*=5.6 Hz), 3.60 (t, 4H, *J*=5.6 Hz).

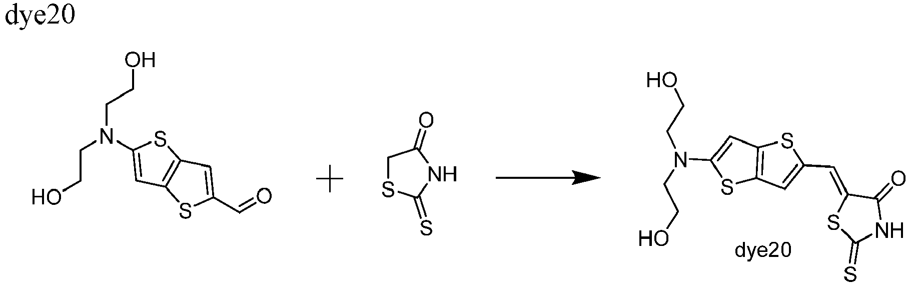

The dye20 was synthesized with reference to the synthetic method of dye1, and the yield was 81.2%. ¹H NMR (400 MHz, DMSO-*d*₆) δ11.15 (s, 1H), 7.64 (s, 1H), 7.57 (s, 1H), 6.59 (s, 1H), 3.85 (t, 4H, *J*=5.6 Hz), 3.60 (t, 4H, *J*=5.6 Hz).

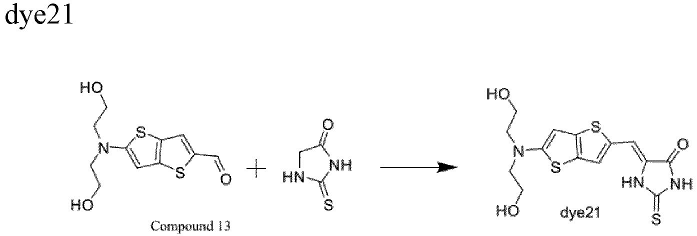

The dye21 was synthesized with reference to the synthetic method of dye1, and the yield was 82.5%. ¹H NMR (400 MHz, DMSO-*d*₆) δ11.35 (s, 1H), 7.64 (s, 1H), 7.57 (s, 1H), 6.59 (s, 1H), 3.85 (t, 4H, *J*=5.6 Hz), 3.60 (t, 4H, *J*=5.6 Hz).

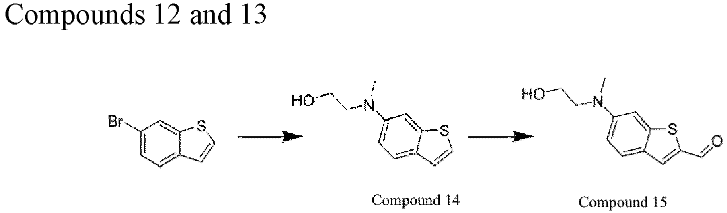

### Compounds 14 and 15

Compounds 14 and 15 were synthesized with reference to the synthetic method of Compounds 7 and 8, and the yield was 50.4%. ¹H-NMR (400 MHz, CDCl₃): δ=9.92 (s, 1H), 7.81 (s, 1H), 7.68 (d, *J*=9.0 Hz, 1H), 6.92 (d, *J*=2.0 Hz, 1H), 6.82 (d, *J*=9.1, 2.3 Hz, 1H), 3.61 (t, *J*=8.0 Hz, 2H), 3.34 (t, *J*=8.0 Hz, 2H), 3.21 (s, 3H).

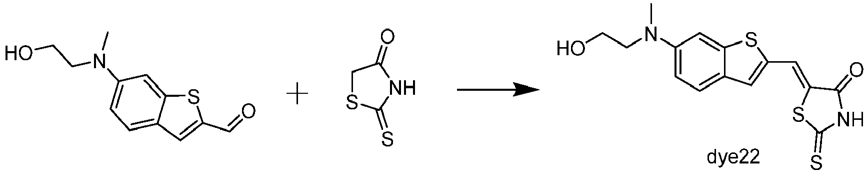

The dye22 was synthesized with reference to the synthetic method of dye1, and the yield was 90.1%. ¹H-NMR (400 MHz, CDCl₃): δ=12.31(s, 1H), 7.86 (s, 1H), 7.81 (s, 1H), 7.68 (d, *J*=9.0 Hz, 1H), 6.92 (d, *J*=2.0 Hz, 1H), 6.82 (d, *J*=9.1, 2.3 Hz, 1H), 3.61 (t, *J*=8.0 Hz, 2H), 3.34 (t, *J*=8.0 Hz, 2H), 3.21 (s, 3H).

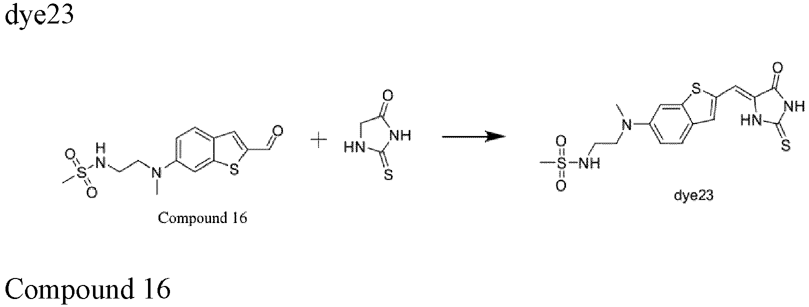

Compound 16 was synthesized with reference to the method disclosed in the following document (Journal of the American Chemical Society, 137(43), 13768-13771; 2015). ¹H-NMR (400 MHz, CDCl₃): δ=10.31 (s, 1H), 7.81 (s, 1H), 7.68 (d, *J*=9.0 Hz, 1H), 6.92 (d, *J*=2.0 Hz, 1H), 6.82 (d, *J*=9.1, 2.3 Hz, 1H), 3.61 (t, *J*=8.0 Hz, 2H), 3.34 (t, *J*=8.0 Hz, 2H), 3.21 (s, 3H), 2.91 (s, 3H).

The dye23 was synthesized with reference to the synthetic method of dye1, and the yield was 79.5%. ¹H-NMR (400 MHz, CDCl₃): δ=8.02 (s, 1H), 7.81 (s, 1H), 7.68 (d, *J*=9.0 Hz, 1H), 6.92 (d, *J*=2.0 Hz, 1H), 6.82 (d, *J*=9.1, 2.3 Hz, 1H), 3.61 (t, *J*=8.0 Hz, 2H), 3.34 (t, *J*=8.0 Hz, 2H), 3.21 (s, 3H), 2.91 (s, 3H).

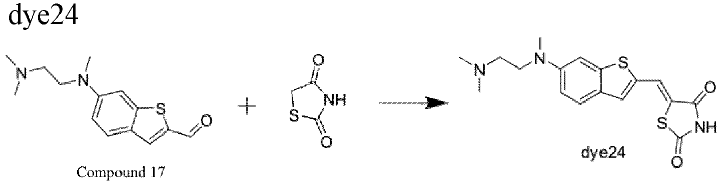

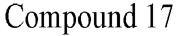

Compound 17 was synthesized with reference to the method disclosed in the following document (Journal of the American Chemical Society, 131(5), 1766-1774; 2009). ¹H-NMR (400 MHz, CDCl₃): δ=10.31 (s, 1H), 7.81 (s, 1H), 7.68 (d, *J*=9.0 Hz, 1H), 6.92 (d, *J*=2.0 Hz, 1H), 6.82 (d, *J*=9.1, 2.3 Hz, 1H), 3.61 (t, *J*=8.0 Hz, 2H), 3.34 (t, *J*=8.0 Hz, 2H), 3.21 (s, 3H), 2.21 (s, 6H).

The dye24 was synthesized with reference to the synthetic method of dye1, and the yield was 68.5%. ¹H-NMR (400 MHz, CDCl₃): δ=12.28 (s, 1H), 8.31 (s, 1H), 7.81 (s, 1H), 7.68 (d, *J*=9.0 Hz, 1H), 6.92 (d, *J*=2.0 Hz, 1H), 6.82 (d, *J*=9.1, 2.3 Hz, 1H), 3.61 (t, *J*=8.0 Hz, 2H), 3.34 (t, *J*=8.0 Hz, 2H), 3.21 (s, 3H), 2.21 (s, 6H).

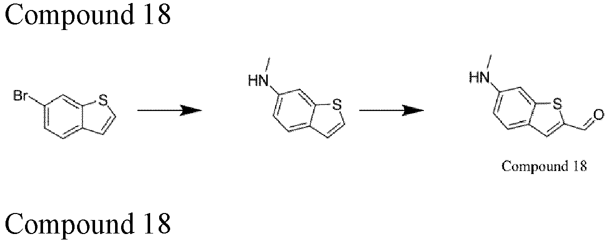

Compound 18 was synthesized with reference to the synthetic method of Compound 15. ¹H-NMR (400 MHz, CDCl₃): δ=10.58 (s, 1H), 7.81 (s, 1H), 7.68 (d, *J*=9.0 Hz, 1H), 6.92 (d, *J*=2.0 Hz, 1H), 6.82 (d, *J*=9.1, 2.3 Hz, 1H), 2.68 (s, 3H).

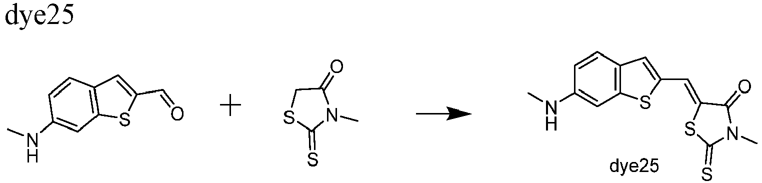

The dye25 was synthesized with reference to the synthetic method of dye1, and the yield was 70.0%. ¹H-NMR (400 MHz, CDCl₃): δ=8.58 (s, 1H), 7.81 (s, 1H), 7.68 (d, *J*=9.0 Hz, 1H), 6.92 (d, *J*=2.0 Hz, 1H), 6.82 (d, *J*=9.1, 2.3 Hz, 1H), 3.21 (s, 3H), 2.68 (s, 3H).

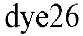

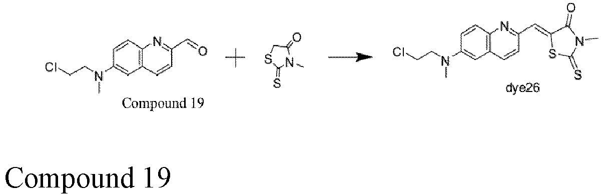

Compound 19 was synthesized with reference to the method disclosed in the following document (Organic Letters, 14(24), 6366-6369; 2012). ¹H-NMR (400 MHz, CDCl₃): δ=10.05 (s, 1H), 7.98 (s, 1H), 7.81 (d, *J*=2.0 Hz, 1H), 7.68 (d, *J*=9.0 Hz, 1H), 6.92 (d, *J*=2.0 Hz, 1H), 6.82 (d, *J*=9.1, 2.3 Hz, 1H), 3.61 (t, *J*=8.0 Hz, 2H), 3.34 (t, *J*=8.0 Hz, 2H), 3.21 (s, 3H).

The dye26 was synthesized with reference to the synthetic method of dye17, and the yield was 70.0%. ¹H-NMR (400 MHz, CDCl₃): δ=8.55 (s, 1H), 7.98 (s, 1H), 7.81 (d, *J*=2.0 Hz, 1H), 7.68 (d, *J*=9.0 Hz, 1H), 6.92 (d, *J*=2.0 Hz, 1H), 6.82 (d, *J*=9.1, 2.3 Hz, 1H), 3.61 (t, *J*=8.0 Hz, 2H), 3.34 (t, *J*=8.0 Hz, 2H), 3.21 (s, 3H), 3.10 (s, 1H).

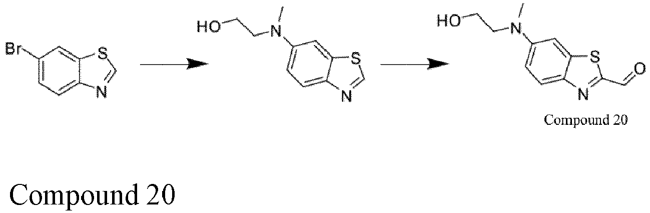

Compound 20 was synthesized with reference to the synthetic method of Compound 8, and the yield was 30.4%. ¹H-NMR (400 MHz, CDCl₃): δ=9.92 (s, 1H), 7.75 (d, *J*=9.0 Hz, 1H), 6.52 (d, *J*=2.0 Hz, 1H), 6.42 (d, *J*=9.1, 2.3 Hz, 1H), 3.61 (t, *J*=8.0 Hz, 3H), 3.34 (t, *J*=8.0 Hz, 3H), 3.21 (s, 3H).

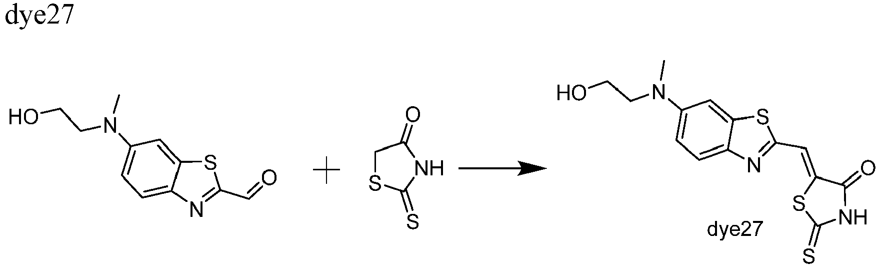

The dye27 was synthesized with reference to the synthetic method of dye1, and the yield was 65.9%. ¹H-NMR (400 MHz, CDCl₃): δ=11.28 (s, 1H), 7.84 (s, 1H), 7.75 (d, *J*=9.0 Hz, 1H), 6.52 (d, *J*=2.0 Hz, 1H), 6.42 (d, *J*=9.1, 2.3 Hz, 1H), 3.61 (t, *J*=8.0 Hz, 3H), 3.34 (t, *J*=8.0 Hz, 3H), 3.21 (s, 3H).

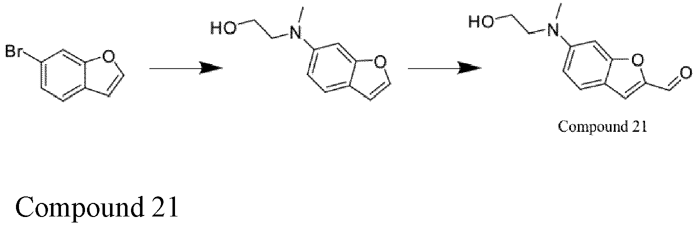

Compound 21 was synthesized with reference to the synthetic method of Compound 8, and the yield was 30.4%. ¹H-NMR (400 MHz, CDCl₃): δ=9.92 (s, 1H), 7.21 (s, 1H), 7.68 (d, *J*=9.0 Hz, 1H), 6.92 (d, *J*=2.0 Hz, 1H), 6.82 (d, *J*=9.1, 2.3 Hz, 1H), 3.61 (t, *J*=8.0 Hz, 2H), 3.34 (t, *J*=8.0 Hz, 2H), 3.21 (s, 3H).

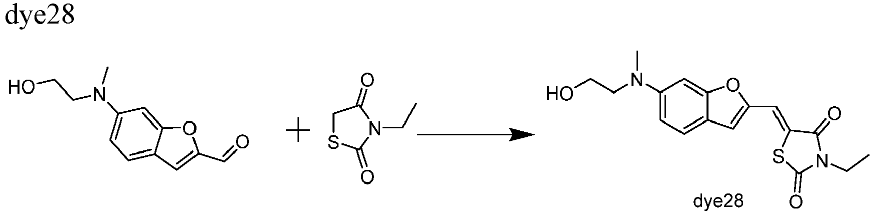

The dye28 was synthesized with reference to the synthetic method of dye1, and the yield was 65.9%. ¹H-NMR (400 MHz, CDCl₃): δ=7.81 (s, 1H), 7.68 (d, *J*=9.0 Hz, 1H), 6.92 (d, *J*=2.0 Hz, 1H), 6.82 (d, *J*=9.1, 2.3 Hz, 1H), 3.61 (t, *J*=8.0 Hz, 3H), 3.60 (q, *J*=5.5 Hz, 2H), 3.34 (t, *J*=8.0 Hz, 3H), 3.21 (s, 3H),1.2 (t, *J*=7.1 Hz 3H).

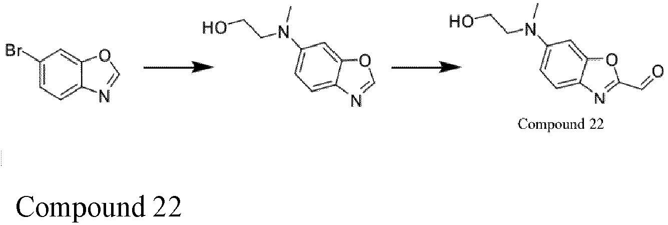

Compound 22 was synthesized with reference to the synthetic method of Compound 8, and the yield was 30.4%. ¹H-NMR (400 MHz, CDCl₃): δ=9.92 (s, 1H), 7.75 (d, *J*=9.0 Hz, 1H), 6.52 (d, *J*=2.0 Hz, 1H), 6.42 (d, *J*=9.1, 2.3 Hz, 1H), 3.61 (t, *J*=8.0 Hz, 3H), 3.34 (t, *J*=8.0 Hz, 3H), 3.21 (s, 3H).

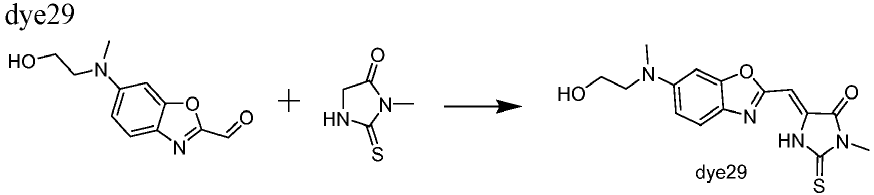

The dye29 was synthesized with reference to the synthetic method of dye1, and the yield was 55.1%. ¹H-NMR (400 MHz, CDCl₃): δ=7.75 (d, *J*=9.0 Hz, 1H), 6.52 (d, *J*=2.0 Hz, 1H), 6.42 (d, *J*=9.1, 2.3 Hz, 1H), 3.61 (t, *J*=8.0 Hz, 3H), 3.34 (t, *J*=8.0 Hz, 3H), 3.21 (s, 3H), 3.10 (s, 3H).

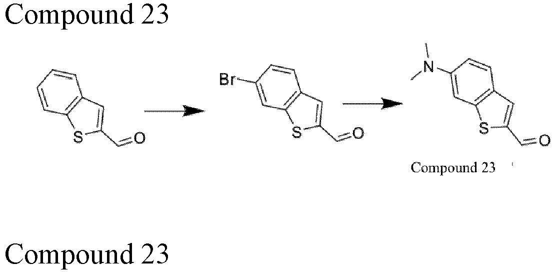

Compound 23 was synthesized with reference to the synthetic method of Compound 11, and the yield was 54.2%. ¹H-NMR (400 MHz, CDCl₃): δ=9.92 (s, 1H), 7.81 (s, 1H), 7.68 (d, *J*=9.0 Hz, 1H), 6.92 (d, *J*=2.0 Hz, 1H), 6.82 (d, *J*=9.1, 2.3 Hz, 1H), 3.21 (s, 6H).

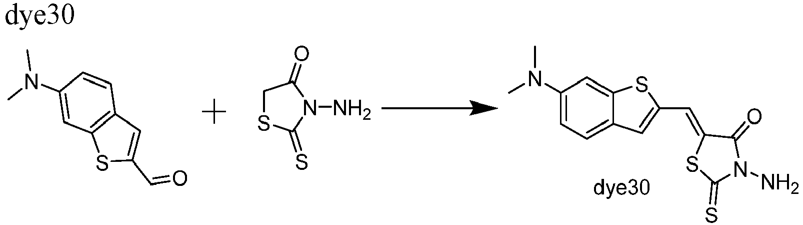

The dye30 was synthesized with reference to the synthetic method of dye1, and the yield was 45.2%. ¹H-NMR (400 MHz, CDCl₃): δ=8.01 (s, 1H), 7.81 (s, 1H), 7.68 (d, *J*=9.0 Hz, 1H), 6.92 (d, *J*=2.0 Hz, 1H), 6.82 (d, *J*=9.1, 2.3 Hz, 1H), 3.21 (s, 6H).

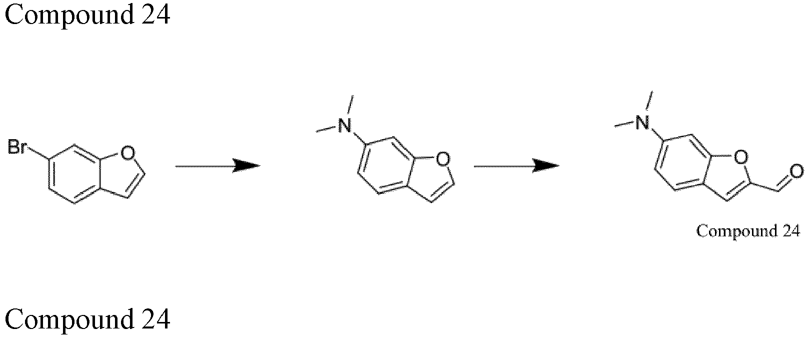

Compound 24 was synthesized with reference to the synthetic method of Compound 11, and the yield was 58.2%. ¹H-NMR (400 MHz, CDCl₃): δ=9.91 (s, 1H), 7.80 (s, 1H), 7.65 (d, *J*=9.1 Hz, 1H), 6.98 (d, *J*=2.1 Hz, 1H), 6.85 (d, *J*=9.0, 2.3 Hz, 1H), 3.21 (s, 6H).

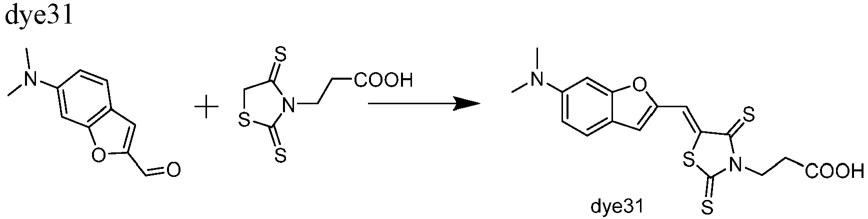

The dye31 was synthesized with reference to the synthetic method of the compound dye1, and the yield was 80.2%. ¹H-NMR (400 MHz, CDCl₃): δ=8.03(s,1H), 7.80 (s, 1H), 7.65 (d, *J*=9.0 Hz, 1H), 6.98 (d, *J*=2.0 Hz, 1H), 6.85 (d, *J*=9.1, 2.3 Hz, 1H), 4.44 (m, 2H), 3.21 (s, 6H) 2.62 (m, 2H).

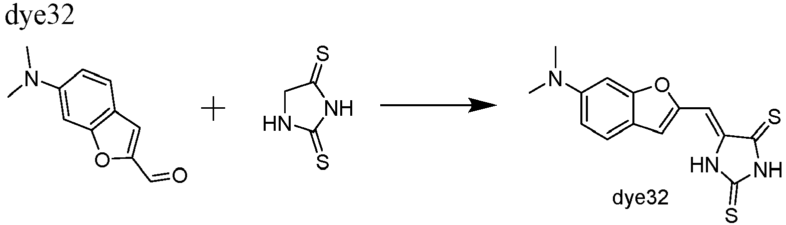

The dye32 was synthesized with reference to the synthetic method of dye1, and the yield was 50.2%. ¹H-NMR (400 MHz, CDCl₃): δ=7.83 (s,1H), 7.80 (s, 1H), 7.65 (d, *J*=9.0 Hz, 1H), 6.98 (d, *J*=2.0 Hz, 1H), 6.85 (d, *J*=9.1, 2.3 Hz, 1H), 3.21 (s, 6H).

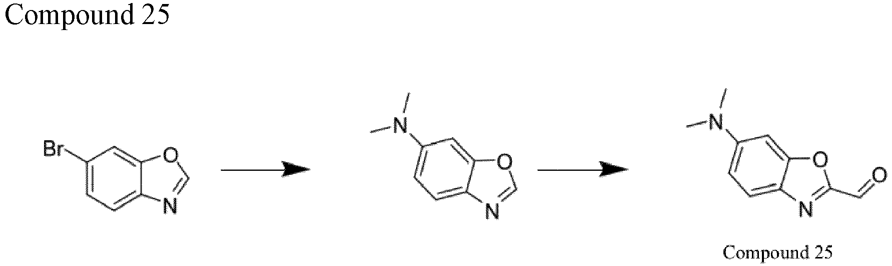

### Compound 25

Compound 25 was synthesized with reference to the synthetic method of Compound 11, and the yield was 45.2%. ¹H-NMR (400 MHz, CDCl₃): δ=7.88 (s, 1H), 7.65 (d, *J*=9.1 Hz, 1H), 6.98 (d, *J*=2.1 Hz, 1H), 6.85 (d, *J*=9.0, 2.3 Hz, 1H), 3.21 (s, 6H).

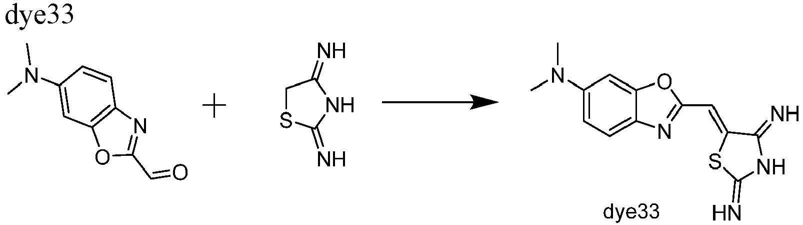

The dye33 was synthesized with reference to the synthetic method of the compound dye1, and the yield was 60.2%. ¹H-NMR (400 MHz, CDCl₃): δ=8.01 (s, 1H), 7.65 (d, *J*=9.1 Hz, 1H), 6.98 (d, *J*=2.1 Hz, 1H), 6.85 (d, *J*=9.0, 2.3 Hz, 1H), 3.21 (s, 6H).

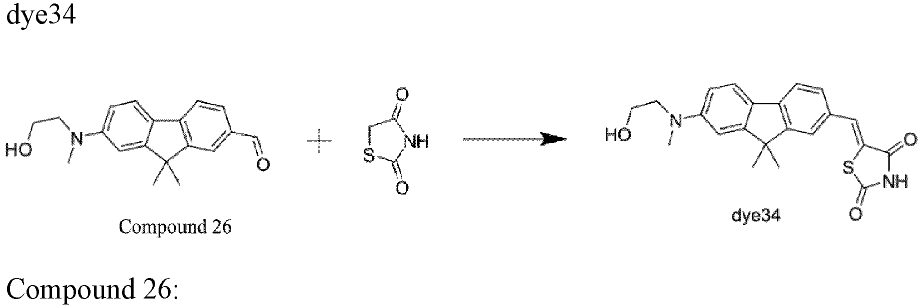

Compound 26 was synthesized with reference to the method disclosed in the following document (Chemistry-A European Journal, 22(30), 10627-10637; 2016). ¹H-NMR (400 MHz, CDCl₃): δ=9.92 (s, 1H), 7.82 (s, 1H), 7.71 (m, 1H), 7.60 (m, 2H), 6.78 (s, 2H), 3.82 (t, 2H, *J*=5.6 Hz), 3.54 (t, 2H, *J*=5.6 Hz), 3.10 (s, 3H), 1.42 (s, 6H).

The dye34 was synthesized with reference to the synthetic method of the compound dye1, and the yield was 50.8%. ¹H-NMR (400 MHz, CDCl₃): δ=11.21 (s, 1H), 7.92 (s, 1H), 7.82 (s, 1H), 7.71 (m, 1H), 7.60 (m, 2H), 6.78 (s, 2H), 3.82 (t, 2H, *J*=5.6 Hz), 3.54 (t, 2H, *J*=5.6 Hz), 3.10 (s, 3H), 1.42 (s, 6H).

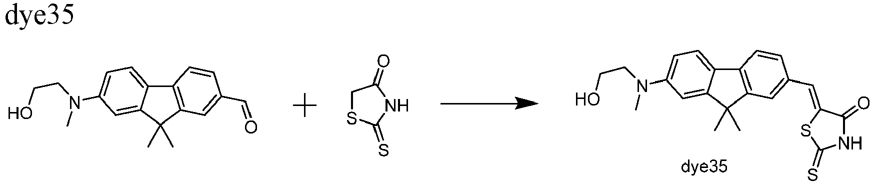

The dye35 was synthesized with reference to the synthetic method of the compound dye1, and the yield was 47.8%. ¹H-NMR (400 MHz, CDCl₃): δ=11.28 (s, 1H), 7.84 (s, 1H), 7.82 (s, 1H), 7.71 (m, 1H), 7.60 (m, 2H), 6.78 (s, 2H), 3.82 (t, 2H, *J*=5.6 Hz), 3.54 (t, 2H, *J*=5.6 Hz), 3.10 (s, 3H), 1.42 (s, 6H).

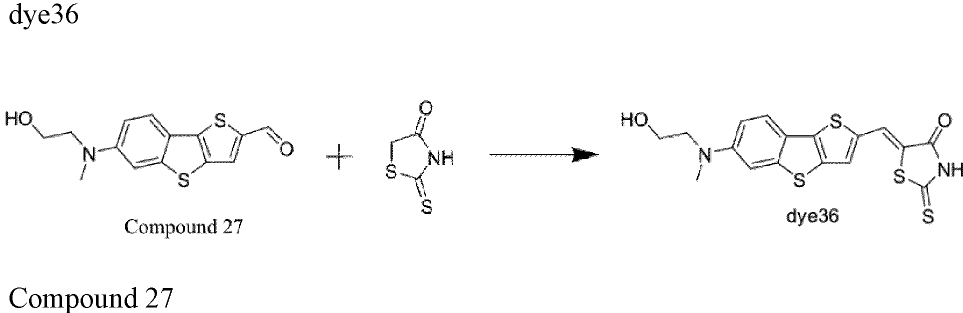

Compound 27 was synthesized with reference to the synthetic method of Compound 26. ¹H NMR (400 MHz, CDCl₃): 10.0 (s, 1H), 8.0 (s, 1H), 7.96-7.98 (t, 1H), 7.89-7.90 (t, 1H), 7.47-7.49 (m, 1H), 3.82 (t, 2H, *J*=5.6 Hz), 3.54 (t, 2H, *J*=5.6 Hz), 3.10 (s, 3H).

The dye36 was synthesized with reference to the synthetic method of the compound dye1, and the yield was 49.7%. ¹H NMR (400 MHz, CDCl₃): 11.28 (s, 1H), 8.04 (s, 1H), 8.0 (s, 1H), 7.96-7.98 (t, 1H), 7.89-7.90 (t, 1H), 7.47-7.49 (m, 1H), 3.82 (t, 2H, J=5.6 Hz), 3.54 (t, 2H, J=5.6 Hz), 3.10 (s, 3H).

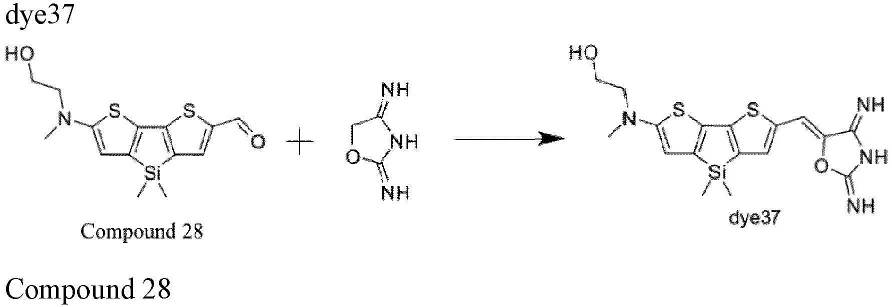

Compound 28 was synthesized with reference to the synthetic method of Compound 26. ¹H-NMR (400 MHz, CDCl₃): δ=9.92 (s, 1H), 7.82 (s, 1H), 7.71 (s, 1H), 3.82 (t, 2H, *J*=5.6 Hz), 3.54 (t, 2H, *J*=5.6 Hz), 3.10 (s, 3H), 1.50 (s, 6H).

The dye37 was synthesized with reference to the synthetic method of the compound dye1, and the yield was 59.6%. ¹H-NMR (400 MHz, CDCl₃): δ=10.28 (s, 1H), 8.05 (s, 1H), 7.82 (s, 1H), 7.71 (s, 1H), 3.82 (t, 2H, *J*=5.6 Hz), 3.54 (t, 2H, *J*=5.6 Hz), 3.10 (s, 3H), 1.50 (s, 6H).

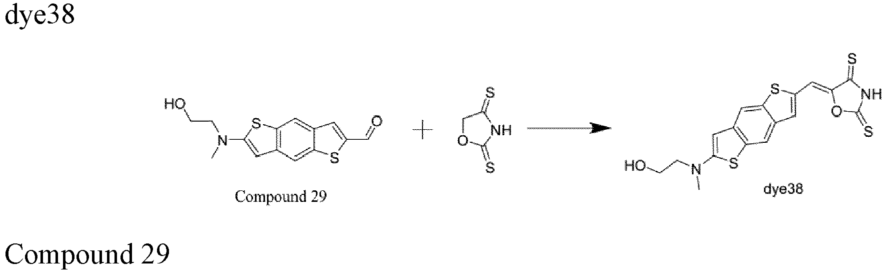

Compound 29 was synthesized with reference to the synthetic method of Compound 26. ¹H-NMR (400 MHz, CDCl₃): δ=9.92 (s, 1H), 7.82 (s, 1H), 7.71 (s, 1H), 7.67 (s, 1H),7.50 (s, 1H), 3.82 (t, 2H, *J*=5.6 Hz), 3.54 (t, 2H, *J*=5.6 Hz), 3.10 (s, 3H), 1.50 (s, 6H).

The dye38 was synthesized with reference to the synthetic method of the compound dye1, and the yield was 49.6%. ¹H-NMR (400 MHz, CDCl₃): δ=11.01 (s, 1H), 8.04 (s, 1H), 7.82 (s, 1H), 7.71 (s, 1H), 7.67 (s, 1H), 7.50 (s, 1H), 3.82 (t, 2H, *J*=5.6 Hz), 3.54 (t, 2H, *J*=5.6 Hz), 3.10 (s, 3H), 1.50 (s, 6H).

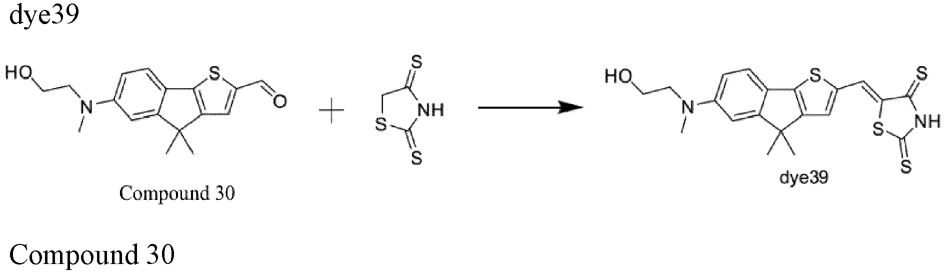

Compound 30 was synthesized with reference to the synthetic method disclosed in the following document (Chemistry of Materials, 31(21), 8810-8819; 2019). ¹H-NMR (400 MHz, DMSO-d₆): δ=10.18 (s, 1H), 7.48 (s, 1H), 7.41 (s, 1H), 7.32 (m, 2H), 3.69 (t, *J*=4.9 Hz, 2H), 3.12 (t, *J*=4.9 Hz, 2H), 3.10 (s, 3H), 1.48 (s, 6H).

The dye39 was synthesized with reference to the synthetic method of the compound dye1, and the yield was 58.2%. ¹H-NMR (400 MHz, DMSO-d₆): δ=14.28 (s, 1H), 7.95(s, 1H), 7.48 (s, 1H), 7.41 (s, 1H), 7.32 (m, 2H), 3.69 (t, *J*=4.9 Hz, 2H), 3.12 (t, *J*=4.9 Hz, 2H), 3.10 (s, 3H), 1.48 (s, 6H).

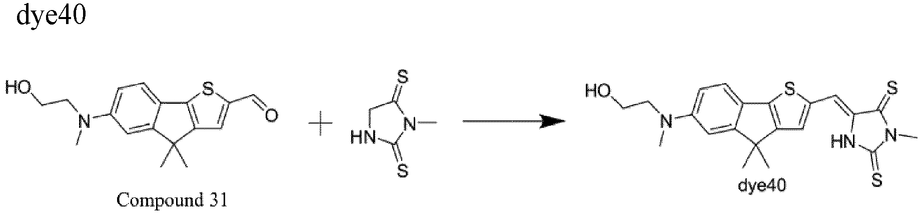

The dye40 was synthesized with reference to the synthetic method of the compound dye1, and the yield was 69.2%. ¹H-NMR (400 MHz, DMSO-d₆): δ=7.95(s, 1H), 7.48 (s, 1H), 7.41 (s, 1H), 7.32 (m, 2H), 3.69 (t, *J*=4.9 Hz, 2H), 3.12 (t, *J*=4.9 Hz, 2H), 3.21 (s, 3H), 3.10 (s, 3H), 1.48 (s, 6H).

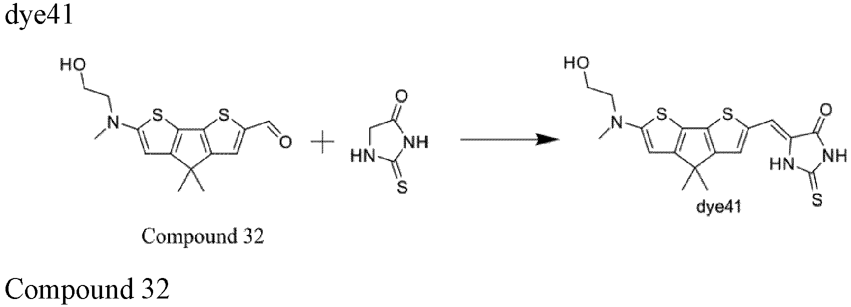

Compound 32 was synthesized with reference to the synthetic method disclosed in the following document (Journal of Organic Chemistry, 73(17), 6587-6594; 2008). ¹H-NMR (400 MHz, DMSO-d₆): δ=10.18 (s, 1H), 7.89 (s, 1H), 7.18 (s, 1H), 3.85 (t, 2H, *J*=5.6 Hz), 3.60 (t, 2H, *J*=5.6 Hz), 3.10 (s, 3H), 1.50 (s, 6H).

The dye41 was synthesized with reference to the synthetic method of the compound dye1, and the yield was 56.6%. ¹H-NMR (400 MHz, DMSO-d₆): δ=12.58 (s, 1H), 8.01 (s, 1H), 7.89 (s, 1H), 7.18 (s, 1H), 3.85 (t, 2H, *J*=5.6 Hz), 3.60 (t, 2H, *J*=5.6 Hz), 3.10 (s, 3H), 1.50 (s, 6H).

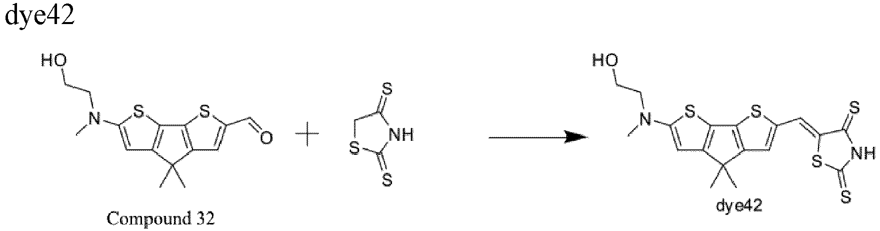

The dye42 was synthesized with reference to the synthetic method of the compound dye1, and the yield was 38.2%. ¹H-NMR (400 MHz, DMSO-d₆): δ=8.01 (s, 1H), 7.89 (s, 1H), 7.18 (s, 1H), 3.85 (t, 2H, *J*=5.6 Hz), 3.60 (t, 2H, *J*=5.6 Hz), 3.10 (s, 3H), 1.50 (s, 6H).

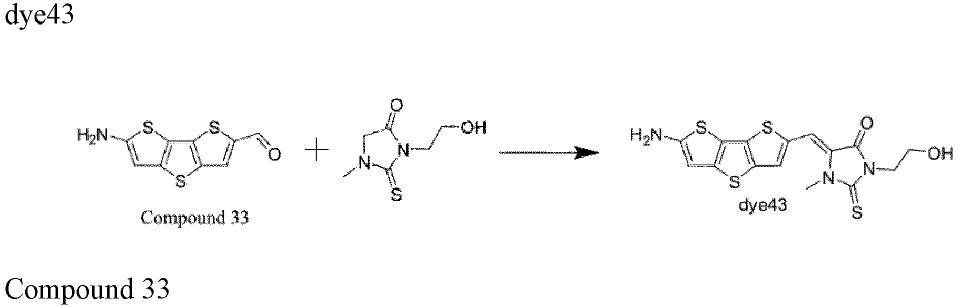

Compound 33 was synthesized with reference to the synthetic method of Compound 26. ¹H-NMR (400 MHz, DMSO-d₆): δ=10.18 (s, 1H), 7.89 (s, 1H), 7.36 (s, 2H), 7.18 (s, 1H), 3.85 (t, 2H, *J*=5.6 Hz), 3.60 (t, 2H, *J*=5.6 Hz) 3.10 (s, 1H).

Dye43 was synthesized with reference to the synthetic method of the compound dye1, and the yield was 78.2%. ¹H-NMR (400 MHz, DMSO-d₆): δ=12.09 (s, 1H), 8.28 (s, 1H), 7.89 (s, 1H), 7.36 (s, 2H), 7.18 (s, 1H), 3.85 (t, 2H, J=5.6 Hz), 3.60 (t, 2H, J=5.6 Hz), 3.10 (s, 1H).

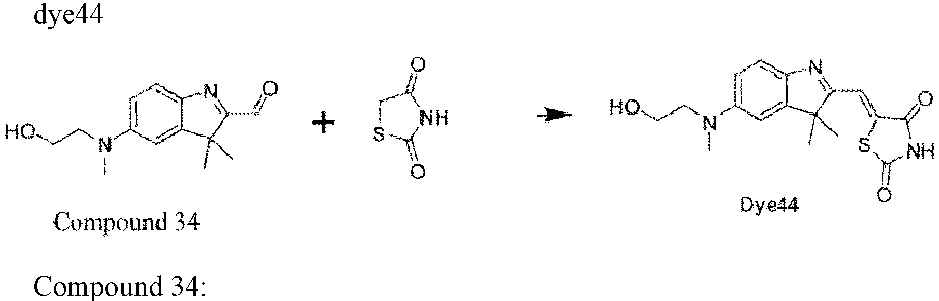

Compound 34 was synthesized with reference to the synthetic method disclosed in the following document (Zhu, Linyong et al, PCT Int. Appl., 2019218876, 21 Nov 2019). ¹H-NMR (400 MHz, CDCl₃): δ=9.74 (s, 1H), 7.76 (d, 1H), 7.60 (s, 1H), 7.03 (d, 1H), 3.85 (t, 2H, *J*=5.6 Hz), 3.60 (t, 2H, *J*=5.6 Hz), 3.10 (s, 3H), 1.42 (s, 6H).

The dye44 was synthesized with reference to the synthetic method of the compound dye1. ¹H-NMR (400 MHz, CDCl₃): δ=11.24 (s, 1H), 8.02 (s, 1H), 7.76 (d, 1H), 7.60 (s, 1H), 7.03 (d, 1H), 3.85 (t, 2H, *J*=5.6 Hz), 3.60 (t, 2H, *J*=5.6 Hz), 3.10 (s, 3H), 1.42 (s, 6H).

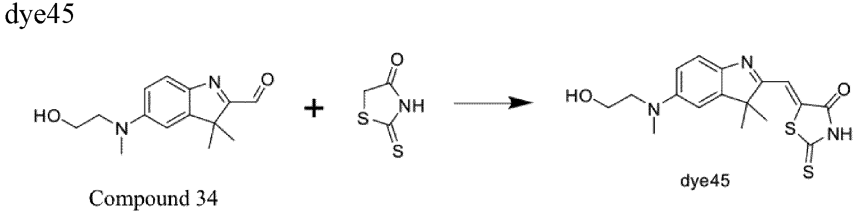

The dye45 was synthesized with reference to the synthetic method of dye1. ¹H-NMR (400 MHz, CDCl₃): δ=13.08 (s, 1H), 8.05 (s, 1H), 7.76 (d, 1H), 7.60 (s, 1H), 7.03 (d, 1H), 3.85 (t, 2H, *J*=5.6 Hz), 3.60 (t, 2H, *J*=5.6 Hz), 3.10 (s, 3H), 1.42 (s, 6H).

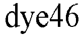

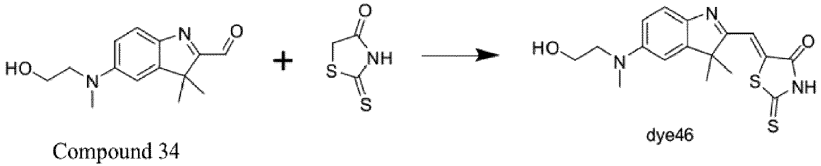

The dye46 was synthesized with reference to the synthetic method of dye1. ¹H-NMR (400 MHz, CDCl₃): δ=15.56 (s, 1H), 8.08 (s, 1H), 7.76 (d, 1H), 7.60 (s, 1H), 7.03 (d, 1H), 3.85 (t, 2H, *J*=5.6 Hz), 3.60 (t, 2H, *J*=5.6 Hz), 3.10 (s, 3H), 1.42 (s, 6H).

### Contrast Examples

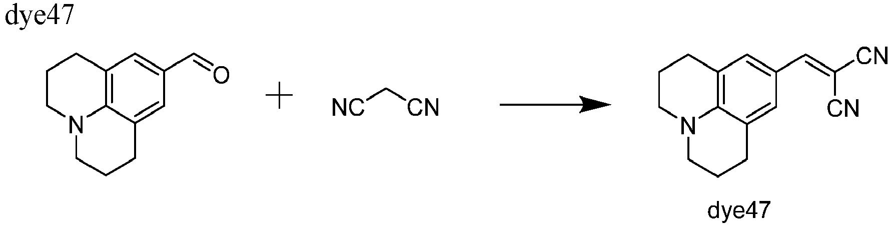

The dye47 was synthesized with reference to the synthetic method disclosed in the following document (European Journal of Organic Chemistry, 2011(25), 4773-4787). ¹H NMR (400 MHz, DMSO-d₆): δ19.21 (s, 1H), 7.31 (s, 1H), 6.95 (s, 2H), 3.25 (dd, *J*=6.6, 4.9 Hz, 4H), 2.68 (t, *J*=6.3 Hz, 4H), 2.05-1.57 (m, 4H).

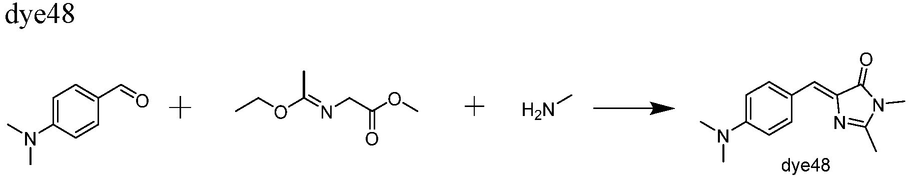

The dye48 was synthesized with reference to the synthetic method disclosed in the following document (J. Am. Chem. Soc. 2020, 142, 17515-17523). ¹H-NMR (400 MHz, Chloroform-d): δ8.07 (d, *J*=8.9 Hz, 2H), 7.11 (s, 1H), 6.70 (d, *J*=8.8 Hz, 2H), 4.29 (s, 3H), 3.05 (s, 6H), 2.31 (s, 3H).

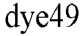

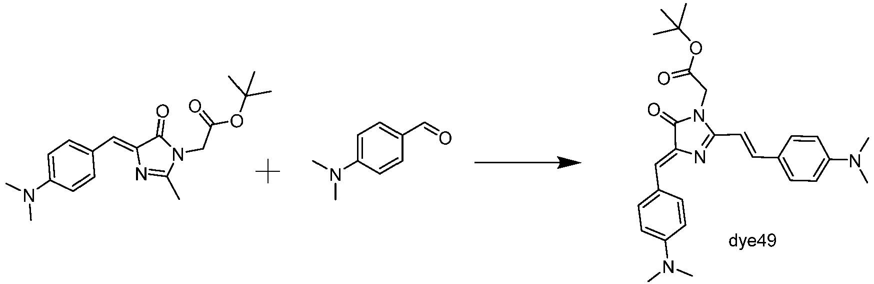

The dye49 was synthesized with reference to the synthetic method disclosed in the following document (J. Am. Chem. Soc. 2020, 142, 17515-17523). ¹H-NMR (400 MHz, Chloroform- d): δ8.19 (d, *J*=8.6 Hz, 2H), 7.97 (d, *J*=15.6 Hz, 1H), 7.48 (d, *J*=8.7, 2H), 7.12 (s, 1H), 6.75 (d, *J*=8.6, 2H), 6.70 (d, *J*=8.6, 2H), 6.42 (d, *J*=15.6 Hz, 1H), 4.45 (s, 2H), 3.07 (s, 6H), 3.05 (s, 6H), 1.49 (m, 9H).

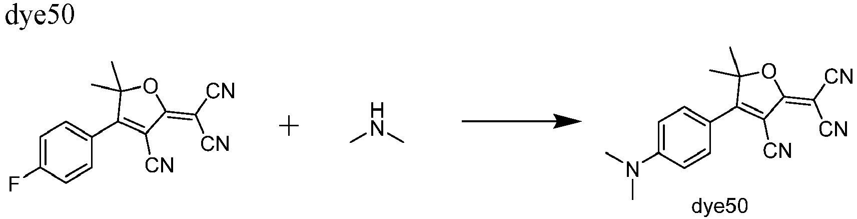

The dye50 was synthesized with reference to the synthetic method disclosed in the following document (Angew. Chem. Int. Ed. 2020, 59,2-10). ¹H-NMR (400 MHz, DMSO-d6): δ8.06 (d, *J*=8.6 Hz, 2H), 6.79 (d, *J*=8.6 Hz, 2H), 3.28 (m, 6H), 1.81 (s, 6H).

### Test Example 1

The fluorescent dye (molecular rotors) prepared in Examples dye1-dye42 were dissolved in DMSO each to prepare a mother solution with a concentration of 1×10⁻² M, and each mother solution was added to glycerol and methanol respectively and was mixed well, and a solution with a final concentration of 1×10⁻⁵ M was prepared. According to different fluorescent dyes, the fluorescence mission spectrum of each fluorescent dye was detected under the same conditions using the maximum excitation wavelength of each fluorescent dye in turn, and the results are shown in Table 1, indicating that the fluorescent dyes of the present disclosure have long-wavelength emission fluorescence, and are sensitive to viscosity changes.

**Table 1**

| Compound | Emission wavelength | Fluorescence intensity ratio in glycerol and methanol |
|---|---|---|
| dye1 | 520 | 695 |
| dye2 | 532 | 587 |
| dye3 | 580 | 637 |
| dye4 | 620 | 984 |
| dye5 | 550 | 1021 |
| dye6 | 608 | 658 |
| dye7 | 620 | 754 |
| dye 8 | 690 | 621 |
| dye9 | 586 | 951 |
| dye10 | 675 | 638 |
| dye11 | 590 | 954 |
| dye12 | 650 | 654 |
| dye13 | 630 | 874 |
| dye14 | 720 | 852 |
| dye15 | 655 | 891 |
| dye16 | 730 | 982 |
| dye17 | 630 | 993 |
| dye18 | 588 | 1026 |
| dye19 | 650 | 1098 |
| dye20 | 650 | 1133 |
| dye21 | 635 | 1098 |
| dye22 | 605 | 687 |
| dye23 | 640 | 985 |
| dye24 | 750 | 465 |
| dye25 | 670 | 626 |
| dye26 | 660 | 871 |
| dye27 | 590 | 765 |
| Dye28 | 690 | 682 |
| dye29 | 650 | 721 |
| dye30 | 640 | 761 |
| dye31 | 685 | 980 |
| dye32 | 654 | 687 |
| dye33 | 622 | 461 |
| dye34 | 680 | 630 |
| dye35 | 740 | 530 |
| dye36 | 735 | 510 |
| dye37 | 720 | 620 |
| dye38 | 730 | 741 |
| dye39 | 820 | 627 |
| dye40 | 730 | 695 |
| dye41 | 735 | 594 |
| dye42 | 800 | 364 |
| dye43 | 720 | 564 |
| dye44 | 680 | 465 |
| dye45 | 717 | 562 |
| dye46 | 745 | 391 |

### Test Example 2

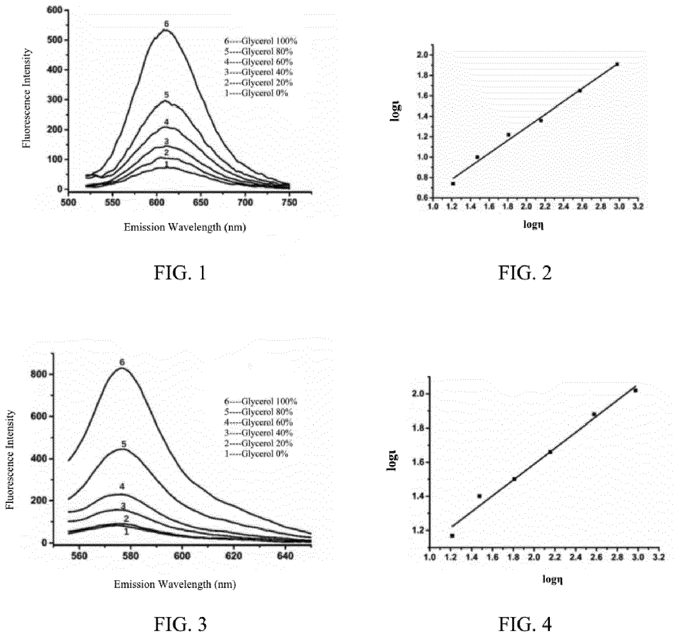

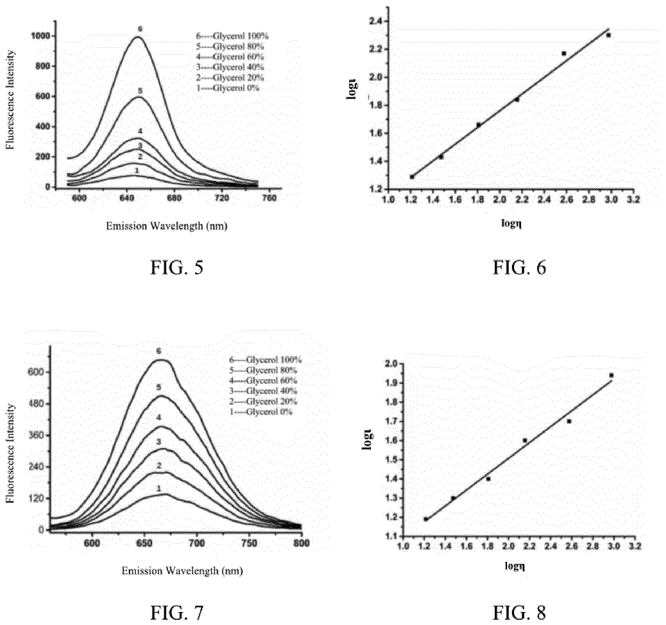

The dye7, dye9, dye12, dye25, dye40 were respectively added into a glycerol-diethanol mixed solution with the following viscosities: 16.4 cp (the volume ratio of glycerol was 0%), 29.8 cp (the volume ratio of glycerol was 20%), 64.5 cp (the volume ratio of glycerol was 40%), 143.5 cp (the volume ratio of glycerol was 60%), 377.0 cp (the volume ratio of glycerol was 80%), 946.0 cp (the volume ratio of glycerol was 100%), so as to prepare solutions with a final concentration of 1×10⁻⁵ M; the fluorescence emission spectra under different viscosity conditions were shown as FIGS. 1, 3, 5, 7 and 9, wherein the emission wavelengths of the above-mentioned molecular rotors were respectively 620 nm, 576 nm, 650 nm, 670 nm and 720 nm. According to the results, fluorescence intensities of fluorescent dyes of the same concentration increase with ambient viscosity. The relationship between the fluorescence intensity log and the solvent intensity log satisfies the Huffman equation, has a fine linear relationship and has a relatively high slope, as shown in FIGS. 2, 4, 6, 8 and 10, proving that the afore-mentioned dyes are sensitive to viscosity and can be used for viscosity testing of unknown samples.

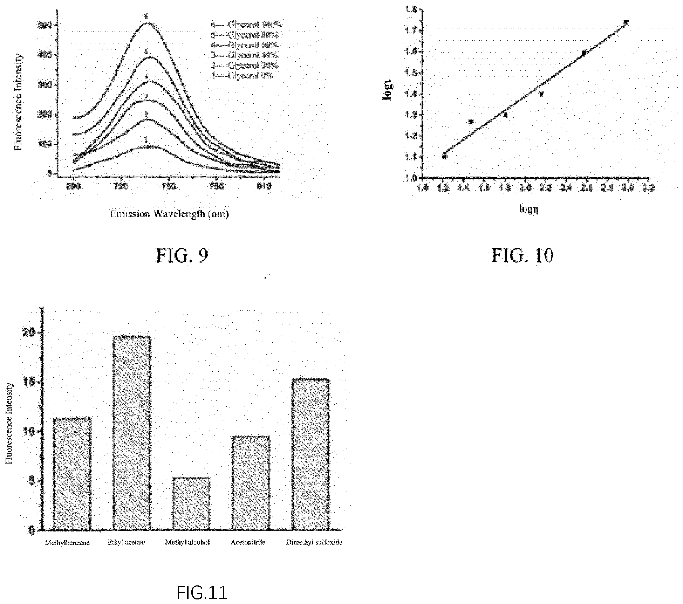

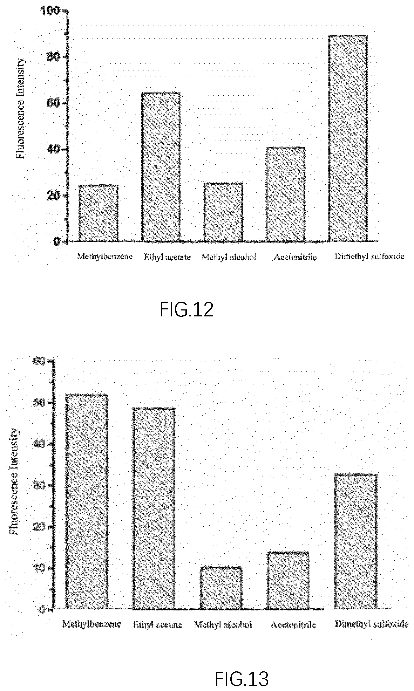

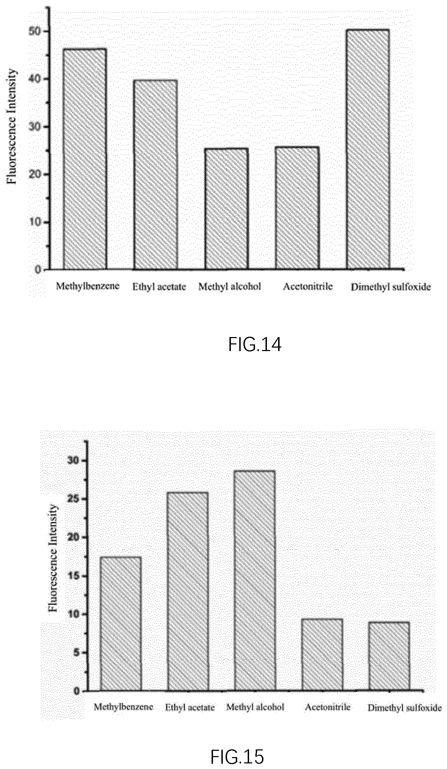

When dye7, dye9, dye12, dye25 and dye40 respectively dissolved into solvents (methylbenzene, ethyl acetate, methyl alcohol, acetonitrile, dimethyl sulfoxide) with different polarities for tests, all of the fluorescence intensities were weak, and would not vary with polarity changes of the solvent, as shown in FIGS. 11, 12, 13, 14 and 15.

The above results indicate that the fluorescent dye in the present disclosure has good specificity for viscosity environment and can reduce non-specific fluorescence caused by polar response.

### Test Example 3

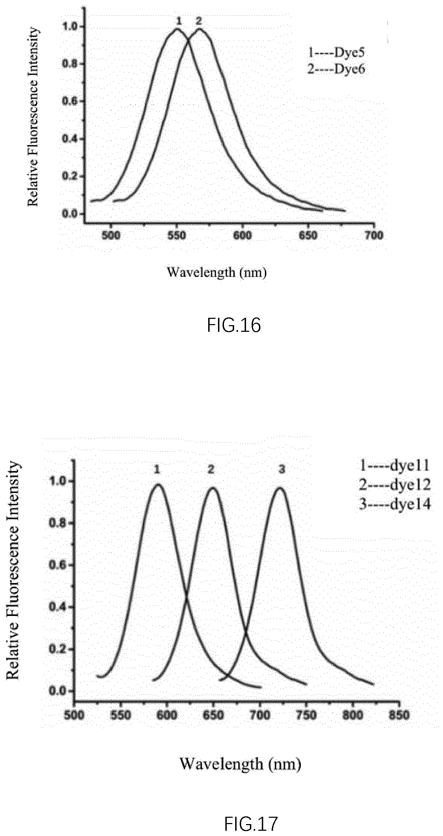

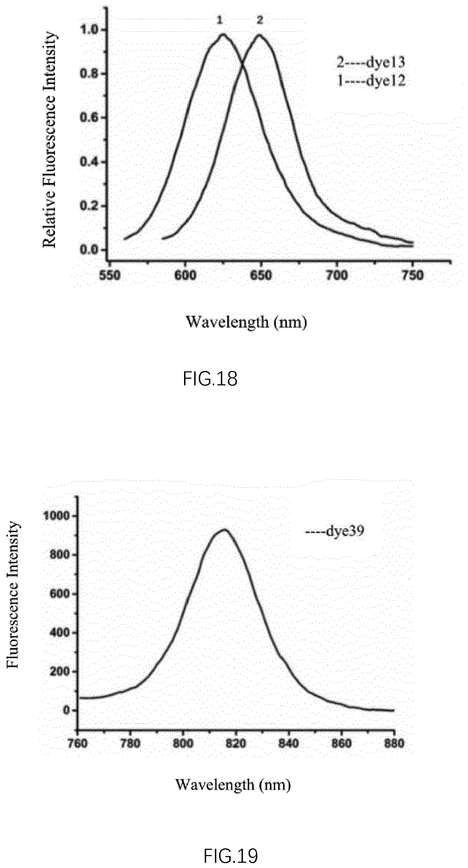

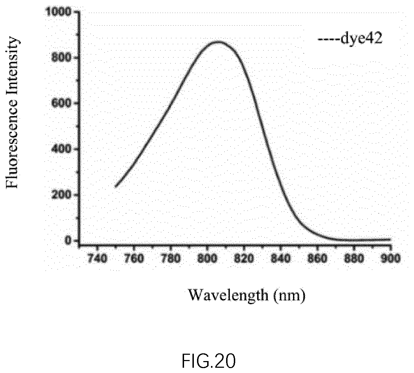

The dye5, dye6, dye11, dye12, dye13, dye14, dye39 and dye42 were added into glycerol to prepare a solution with a final concentration of 1×10⁻⁵ M, fluorescent dyes were excited with corresponding maximum excitation wavelength so as to obtain the emission spectra, and some of the emission spectra were normalized. The emission spectra of dye5 and dye6 were compared, as shown in FIG. 16, the wavelength increased by about 50 nm when oxo-carbonyl group of the electron-pulling part turned into thiocarbonyl; the emission spectra of dye11, dye12 and dye14 were compared, as shown in FIG. 17, indicating that the effects of increasing the wavelength can be superimposed; the emission spectra of dye12 and dye13 were compared, as shown in FIG. 18, and the wavelength was shortened by 20 nm when sulfur on the five-membered ring of the electron-pulling part turned into a nitrogen atom, proving that the emission wavelengths of the afore-mentioned dyes can be easily realized by changing one atom in the structure, as a result, adjustable spectra are possible while it is ensured that the structures of dyes do not change much (water solubility and permeability of the membrane did not change much), and, by means of this wavelength adjustment mechanism, the maximum emission wavelength of a dye can be over 800, as shown in FIGS. 19 and 20, which is very advantageous for dyes used in live imaging.

### Test Example 4

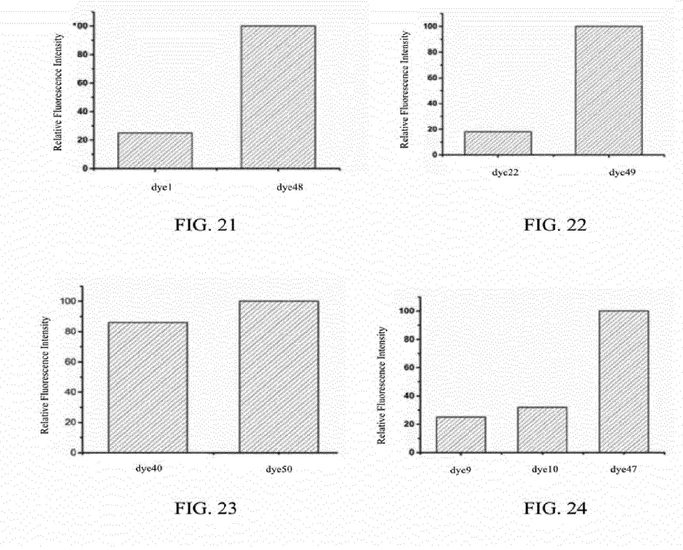

The dye1 and dye48; dye22 and dye49; dye40 and dye50; dye9, dye10 and dye47 were added to PBS to prepare a solution with a final concentration of 1×10⁻⁵ M, corresponding maximum excitation wavelengths were used for exciting the dyes so that their fluorescence intensities in PBS could be detected, and normalization was performed with the strongest fluorescence intensity in each group of samples as 100, as shown respectively in FIGS. 21, 22, 23 and 24. According to the results, relative to fluorescent dyes disclosed in the documents, the fluorescent dye of the present disclosure have a special structure of electron-pulling group, which enables the fluorescent dye of the present disclosure to have a lower background fluorescence.

### Test Example 5

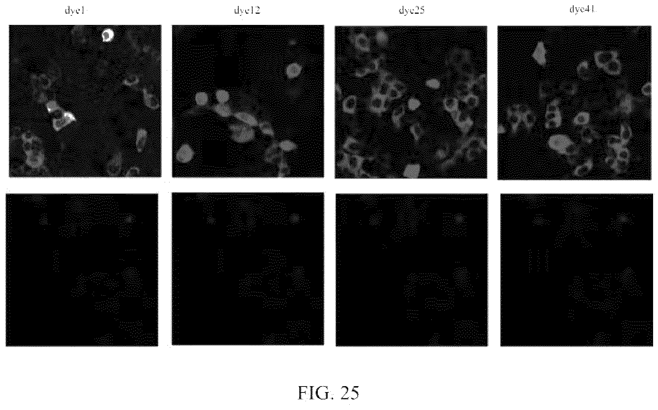

Stable cell line (293T/17) continuously expressing RNA aptamer (UUGCCAUGUGUAUGUGGGUUCGCCCACAUACUCUGAUGAUCCGGUG UAGUAGGUCCGGUGUAGUAGGUCCGGUGUAGUAGGUCCGGUGUAGU AGGUCCUUCGGGACCGGAAGAAUUGAUCUCGGCCGGACCGGAAGAAU UGAUCUCGGCCGGACCGGAAGAAUUGAUCUCGGCCGGACCGGAAGAA UUGAUCUCGGCCGGAUCAUUCAUGGCAA) and control cells (293T/17) were used, and cell culture media of dye1, dye12, dye25, and dye41 with a final concentration of 2 uM substituted original cell culture media after the cell line and control cells grew to a cell confluence of 90% under the conventional mammalian cell culture condition (37°C, 5% carbon dioxide, 100% relative humidity), and were incubated for 10 min (37°C, 5% carbon dioxide, 100% relative humidity). Corresponding wavelengths were observed with a confocal microscopy using dyes, wherein: the dye basically did not fluoresce before binding, but the fluorescence was notably activated after binding; moreover, the compound could also bind to proteins even in the cells, as shown in FIG. 25, sells expressing aptamers emit bright fluorescence, while cells not expressing aptamers do not, which indicates that the dye of the present disclosure is excellent in membrane permeability and can be used for labeling living cell aptamers.

### Test Example 6

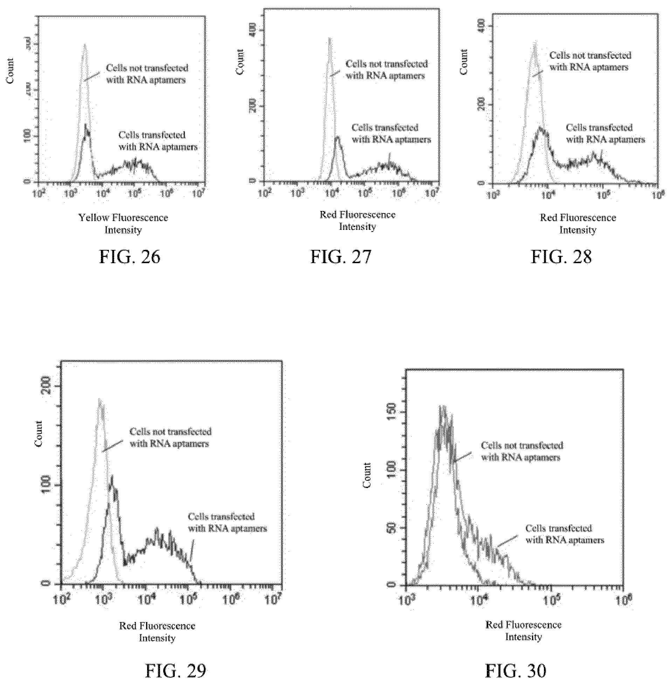

Stable cell line (293T/17) continuously expressing RNA aptamer (UUGCCAUGUGUAUGUGGGUUCGCCCACAUACUCUGAUGAUCCGGUG UAGUAGGUCCGGUGUAGUAGGUCCGGUGUAGUAGGUCCGGUGUAGU AGGUCCUUCGGGACCGGAAGAAUUGAUCUCGGCCGGACCGGAAGAAU UGAUCUCGGCCGGACCGGAAGAAUUGAUCUCGGCCGGACCGGAAGAA UUGAUCUCGGCCGGAUCAUUCAUGGCAA) and control cells (293T/17) were used, and the cells were digested after the cell line and control cells grew to a cell confluence of 90% under the conventional mammalian cell culture conditions (37°C, 5% carbon dioxide, 100% relative humidity), and were centrifuged at 800 rpm, and then the cells were re-suspended with PBS containing 1 µM of dye1, dye12, dye25, dye41 molecules, and were incubated for 5 minutes before flow detection, see flow diagrams 26 to 29 for the detection results, wherein: dye3, dye13, dye21 and dye34 had very low dye background before binding, but their fluorescence was notably activated after binding, and the dyes could also bind to RNA aptamers even in cells and emit bright fluorescence; there was basically no non-specific fluorescence activation, indicating that the fluorescent dye of the present disclosure has very low background fluorescence and very low non-specific fluorescence in cells, and can be used for labeling biomacromolecules with low abundance, such as nucleic acid; however, the background fluorescence of dye50 is obvious, as shown in FIG. 30, and thus can hardly be used as a marker for biomacromolecules with low abundance, such as nucleic acids.

## Claims

1. A fluorescent dye, comprising: an electron donor part D, a conjugate system E and an electron acceptor part A, wherein the fluorescent dye is represented by Formula (I) 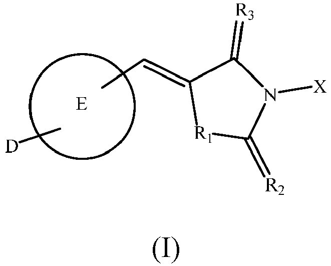
the electron donor part-D is (X₁)(X₂)N-, X₁ and X₂ are independently selected from hydrogen, alkyl and modified alkyl; X₁ and X₂ are optionally interconnected, and form an aliphatic heterocycle with N atoms;
the conjugate system E is formed by at least one conjugate connection selected from a sub-aromatic ring and a sub-aromatic heterocycle, wherein each hydrogen atom contained in the conjugate system E is optionally and independently substituted by alkyl groups;
X₁ and X₂ in the electron donor part D- optionally and independently form, together with N atoms, an aliphatic heterocycle with the conjugate system E;
the electron acceptor part optionally forms a ring structure represented by Formula (I-1): 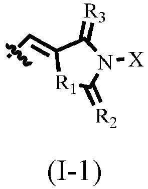 wherein:
R₁ is independently selected from -O-, -S- and -(NRₐ)-, and Rₐ is selected from hydrogen and alkyl;
R₂ is independently selected from =O, =S, and =NH;
R₃ is independently selected from =O, =S and =NH; and
X is independently selected from hydrogen, alkyl or modified alkyl;
wherein: the "alkyl" is C₁-C₃₀ straight or branched alkyl; preferably, the "alkyl" is C₁-C₁₀ straight or branched alkyl; preferably, the "alkyl" is C₁-C₇ straight or branched alkyl; preferably, the "alkyl" is C₁-C₅ straight or branched alkyl; preferably, the "alkyl" is selected from methyl, ethyl, n-propyl, isopropyl, n-butyl, isobutyl, tert-butyl, sec-butyl, n-pentyl, 1-methylbutyl, 2-methylbutyl, 3-methylbutyl, isopentyl, 1-ethylpropyl, neopentyl, n-hexyl, 1-methylpentyl, 2-methylpentyl, 3-methylpentyl, isohexyl, 1,1-dimethylbutyl, 2,2-dimethylbutyl, 3,3-dimethylbutyl, 1,2-dimethylbutyl, 1,3-dimethylbutyl, 2,3-dimethylbutyl, 2-ethylbutyl, n-heptyl, 2-methylhexyl, 3-methylhexyl, 2,2-dimethylpentyl, 3,3-dimethylpentyl, 2,3-dimethylpentyl, 2,4-dimethylpentyl, 3-ethylpentyl or 2,2,3-trimethylbutyl;
the "modified alkyl" is a group obtained by replacing any carbon atom in the alkyl with at least one group selected from halogen atom, -O-, -OH, -CO-, -SO₂-, -(S=O)-, primary amino group, secondary amino group and tertiary amino group, and the modified alkyl has 1 to 30 carbon atoms (optionally, the modified alkyl has 1 to 12 carbon atoms; optionally, the modified alkyl has 1 to 10 carbon atoms; optionally, the modified alkyl has 1 to 8 carbon atoms; optionally, the modified alkyl has 1 to 5 carbon atoms; optionally, the modified alkyl has 1 to 2 carbon atoms),
the replacement of carbon atoms refers to that carbon atoms or the carbon atoms and hydrogen atoms thereon together are replaced by corresponding groups;
the "aliphatic heterocycle" is a saturated or unsaturated 4- to 10-membered monocyclic or polycyclic aliphatic heterocycle containing at least one heteroatom of N, O or S on the ring, and when the aliphatic heterocycle contains an S atom, it is -S-, -SO- or -SO₂-; the aliphatic heterocycle is optionally substituted by alkyl;
the "sub-aromatic ring" is a 6- to 13-membered monocyclic or fused dicyclic or fused polycyclic sub-aromatic group;
the "sub-aromatic heterocycle" is a 5- to 12-membered monocyclic or fused dicyclic or fused polycyclic sub-aromatic heterocyclic group containing at least one heteroatom selected from N, O, S or Si on the ring;
the "halogen atom" is respectively and independently selected from F, Cl, Br and I;
the "primary amino group" is an RNH₂ group;
the "secondary amino group" is an RNHR' group;
the "tertiary amino group" is an RNR'R" group;
each R, R' and R" respectively and independently is a single bond, alkyl, alkylene, modified alkyl or modified alkylene;
the "alkylene" is a C₁-C₁₀ straight or branched alkylene, and optionally is a C₁-C₆ straight or branched alkylene;
the "modified alkylene" is a group obtained by replacing any carbon atom of C₁-C₁₀ (optionally C₁-C₆) alkyl or alkylene with a group selected from -O-, -OH, -CO-, -CS- and -(S=O)-; the replacement of carbon atoms refers to that carbon atoms or the carbon atoms and hydrogen atoms thereon together are replaced by corresponding groups.

2. The fluorescent dye according to claim 1, wherein: the modified alkyl or modified alkylene respectively and independently is a group containing at least one group selected from -OH, -O-, -NH₂, ethylene glycol unit (-(CHzCHzO)n-), C₁-C₈ alkyl, C₁-C₈ haloalkyl (preferably C₂-C₆ haloalkyl), C₁-C₈ alkyl carboxyl (preferably C₂-C₆ alkyl carboxyl), -SO₂-NH-, halogen atom, cyano group and nitryl, wherein n is 1 to 30, more preferably 1 to 10; and more preferably 1 to 2;
optionally, the C₁-C₈ alkyl is methyl, ethyl, propyl or isopropyl; the C₁-C₈ alkoxy is methoxy, ethoxy, propoxy or isopropoxy, the C₁-C₈ acyl oxygroup is acetoxyl group, ethyl, propyl or isopropyl, and the C₁-C₈ haloalkyl is trifluoromethyl, chloromethyl or bromomethyl;
optionally, the aliphatic heterocycle is selected from morpholine, thiomorpholine and tetrahydropyridine; and
optionally, X₁ and X₂ respectively and independently are C₁₋₁₀ straight or branched alkyl substituted by one or more groups selected from hydroxyl, cyano, halogen and carboxyl.

3. The fluorescent dye according to claim 1 or 2, wherein: the conjugate system E is a structure selected from following Formulae (I-2-1) to (I-2-20): 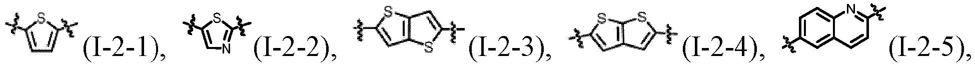 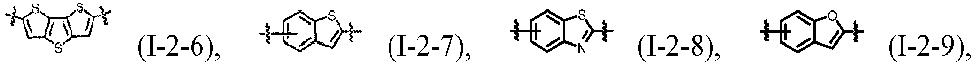 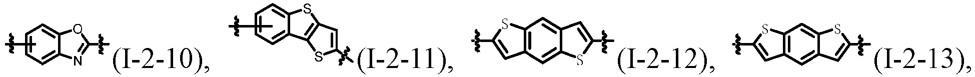 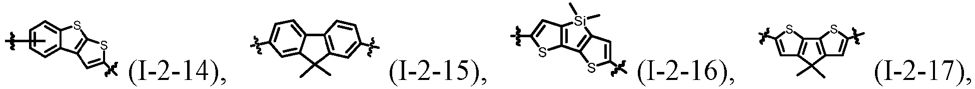 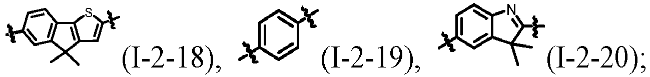
or, the conjugate system E and (X₁)(X₂)N- form a aliphatic heterocycle as represented by Formulae (I-3-1) to (I-3-4): 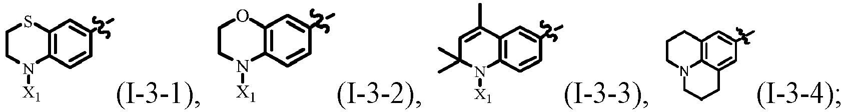
optionally, the electron acceptor part A is one selected from Formulae (I-4-1) to (I-4-43):

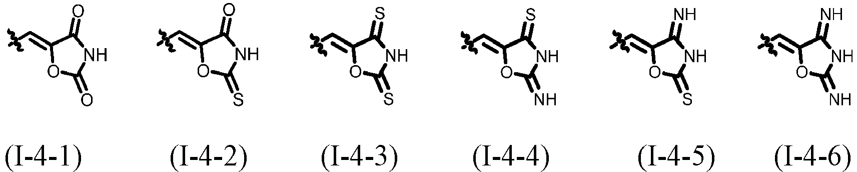

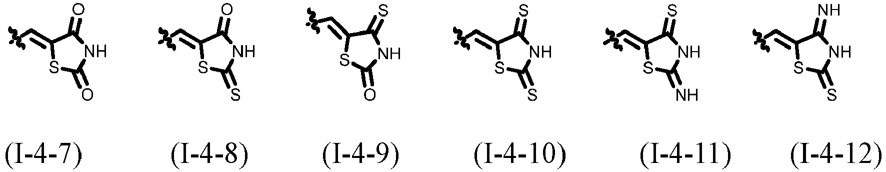

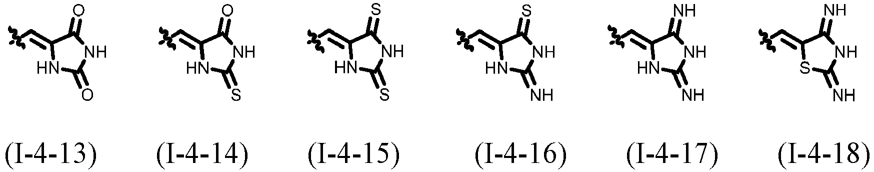

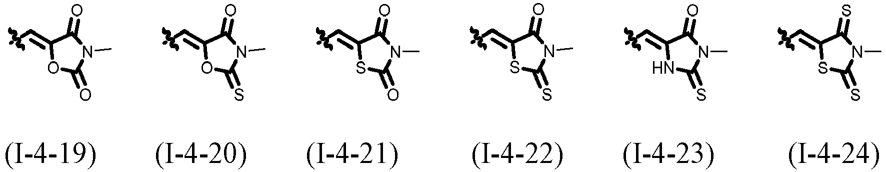

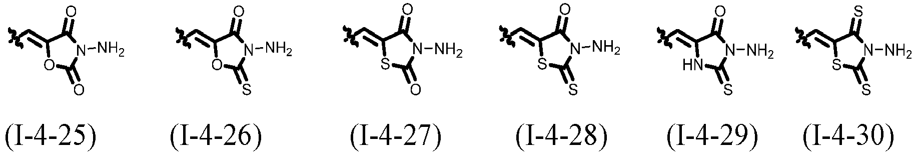

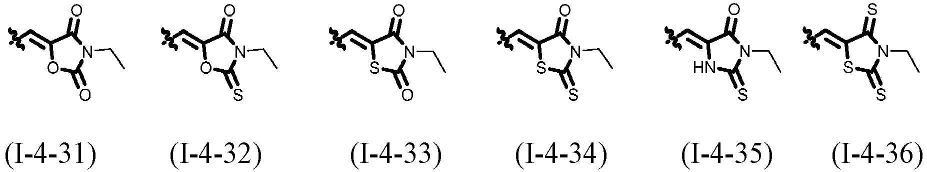

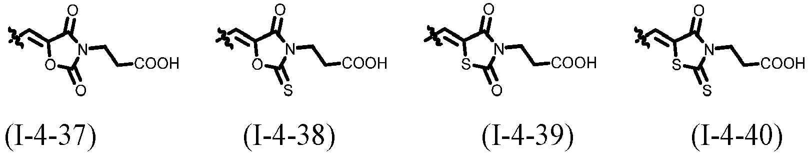

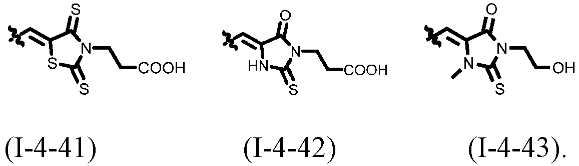

4. The fluorescent dye according to any one of claims 1 to 3, wherein: the fluorescent dye represented by formula (I) is selected from the compounds represented by the formulae below:

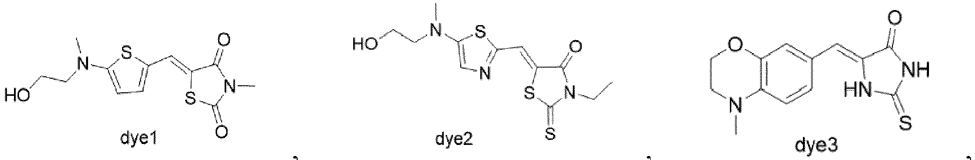

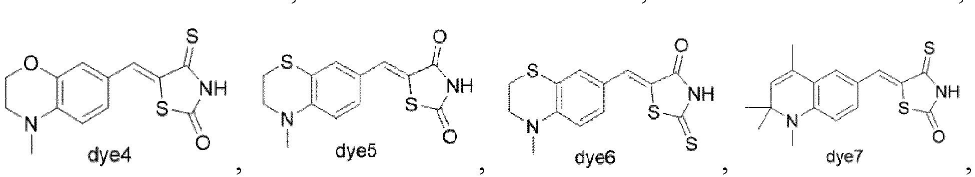

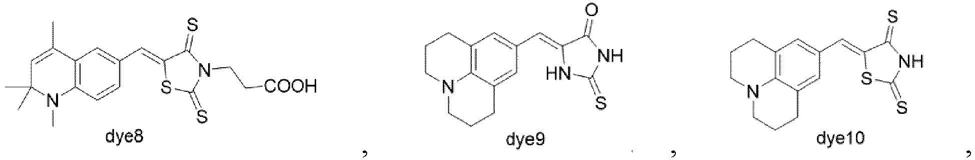

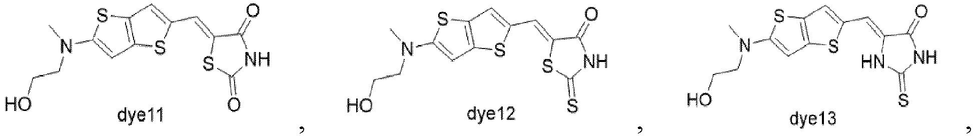

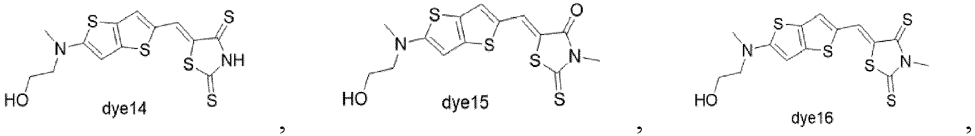

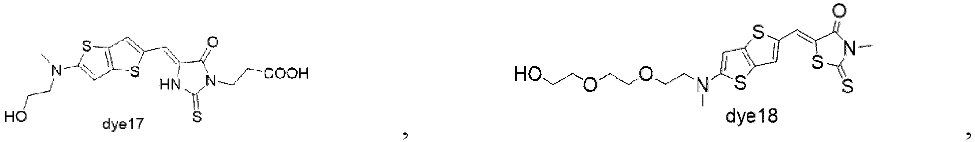

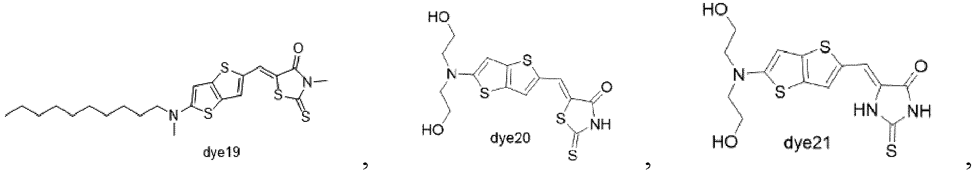

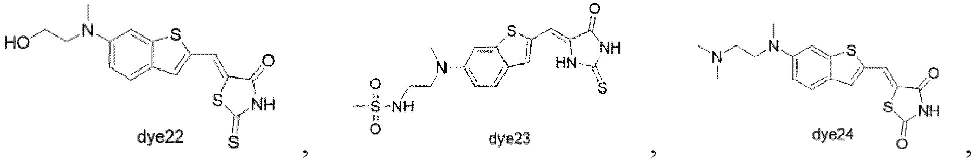

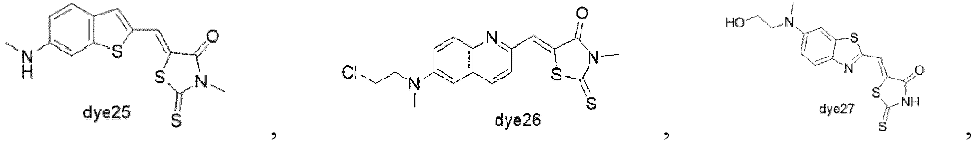

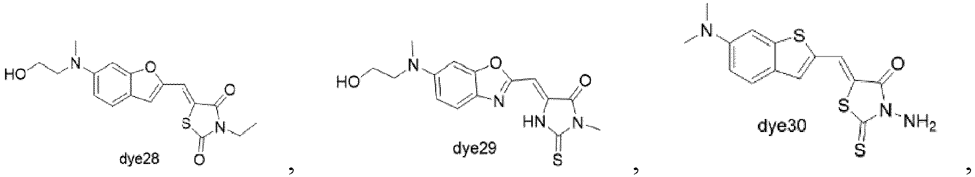

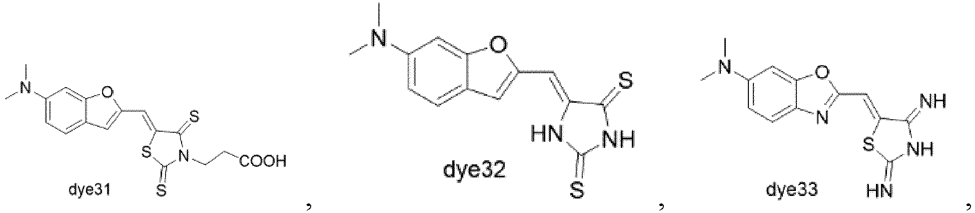

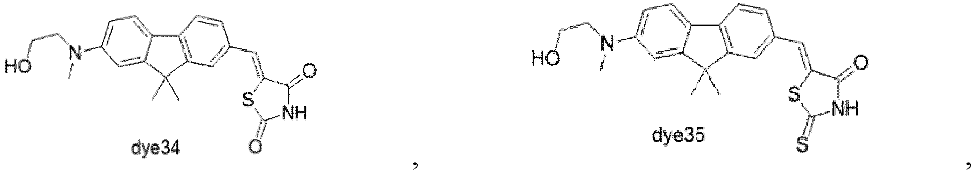

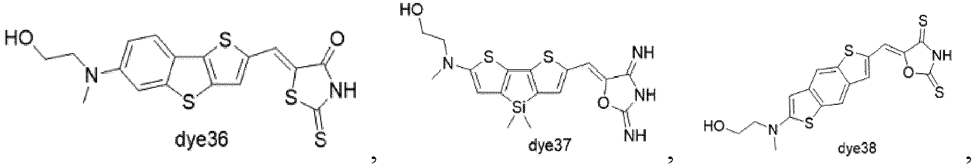

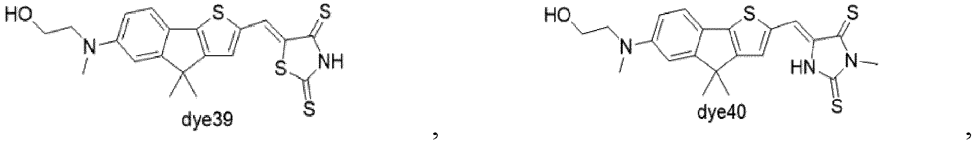

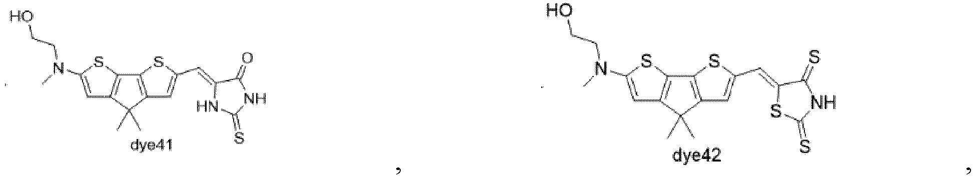

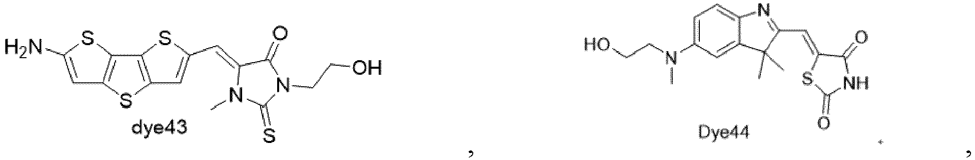

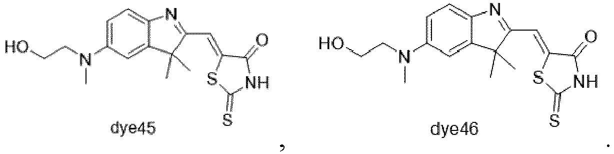

5. A method for preparing the fluorescent dye according to any one of claims 1 to 4, including a synthetic reaction step of producing the fluorescent dye represented by Formula (I) via reaction of a compound represented by Formula (a) with a compound represented by Formula (b):

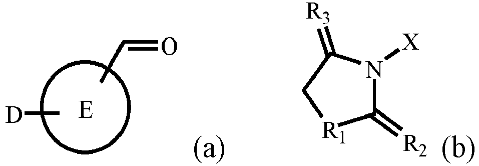

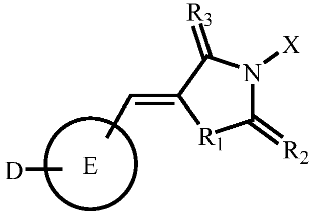

6. Use of the fluorescent dye according to any one of claims 1 to 4 in viscosity testing, protein fluorescent labeling, nucleic acid fluorescent labeling, protein quantification or detection, or nucleic acid quantification or detection.

7. Use of the fluorescent dye according to any one of claims 1 to 4 in preparing reagents for viscosity testing, protein fluorescent labeling, nucleic acid fluorescent labeling, protein quantification or detection, or nucleic acid quantification or detection.

8. A fluorescent activated and lighted probe, comprising the fluorescent dye according to any one of claims 1 to 4.

9. Use of the fluorescent activated and lighted probe according to claim 8 in protein fluorescent labeling, nucleic acid fluorescent labeling, protein quantification or detection, or nucleic acid quantification or detection.

10. Use of the fluorescent activated and lighted probe according to claim 8 in preparing reagents for protein fluorescent labeling, nucleic acid fluorescent labeling, protein quantification or detection, or nucleic acid quantification or detection.
